# EUROPEAN PATENT APPLICATION

(11) **EP 4 450 505 A1**
(43) Date of publication of application: **23.10.2024**
(21) Application number: 22907977.7
(22) Date of filing: 15.12.2022
(51) Int. Cl.: C07D 487/08, A61K 31/519, A61P 25/28, A23L 33/10

(54) **MACROCYCLIC PYRIMIDINE DERIVATIVE, METHOD FOR PREPARING SAME, AND PHARMACEUTICAL COMPOSITION FOR PREVENTION OR TREATMENT OF NEURODEGENERATIVE DISEASE CONTAINING SAME AS ACTIVE INGREDIENT**

(30) Priority: 15.12.2021 KR 20210179316; 09.12.2022 KR 20220171787
(71) Applicant: Whan In Pharmaceutical Co., Ltd., Songpa-gu, Seoul 05855 (KR)
(72) Inventor: PARK, Hwang-Seo, Seoul 04731 (KR); SHIN, Ho-Chul, Seoul 05855 (KR); YE, In-Hae, Seoul 05855 (KR); LEE, Kyu-Hwan, Seoul 05855 (KR); HAN, Song-Hee, Seoul 05855 (KR); YOU, Byoung-Hoon, Ansan-si, Gyeonggi-do 15624 (KR); SEO, Jee-Yeon, Seoul 05855 (KR); HEO, Jae-Kyung, Suwon-si, Gyeonggi-do 16530 (KR)
(74) Representative: Abel & Imray LLP
(86) International application number: PCT/KR2022/020504
(87) International publication number: WO 2023/113510

(57) **Abstract**

The present invention relates to a macrocyclic pyrimidine derivative, a method for preparing the same, and a pharmaceutical composition for the prevention or treatment of a neurodegenerative disease, containing the same as an active ingredient. The macrocyclic pyrimidine derivative according to the present invention has excellent LRRK2 inhibitory activity and efficiently penetrates the blood-brain barrier (BBB), and thus can be effectively used as a pharmaceutical composition for the prevention or treatment of a neurodegenerative disease.

## Description

### TECHNICAL FIELD

The present invention relates to a macrocyclic pyrimidine derivative, a method for preparing the same, and a pharmaceutical composition containing the same as an effective ingredient for preventing or treating neurodegenerative disease.

### BACKGROUND ART

Neurodegenerative disease is a brain disease that occurs with age and is known to be caused by the accumulation of environmental and genetic factors. This is a general term for a disease that causes various symptoms as degenerative changes occur due to gradual and steady death of nerve cells in the central nervous system, and is characterized by loss of neural structure and function, including cell damage and memory impairment.

Representative examples include Parkinson's disease, Alzheimer's disease, Huntington's disease, Pick's disease, amyotrophic lateral sclerosis, prion disease, motor neuron disease, spinocerebellar ataxia, spinal muscular atrophy, Creutzfeldt-Jakob disease, alcohol related dementia, etc.

Parkinson's disease is one of the representative neurodegenerative diseases along with Alzheimer's disease, and is caused by a lack of dopamine, a neurotransmitter, due to the loss of dopaminergic neurons in the substantia nigra of the brainstem. As a result, protein aggregates called Lewy bodies are formed within nerve cells, causing motor dysfunction such as stooped posture, tremors, stiffness, and instability, as well as mental symptoms such as depression, anxiety, and sleep disorders.

The cause of Parkinson's disease is not clearly known, but this is thought to be caused by a combination of factors, including environmental factors, genetic factors, aging, mitochondrial dysfunction, and abnormalities in the ability to process unnecessary proteins. Most Parkinson's disease occurs sporadically, but 5 to 10% are known to be hereditary diseases caused by genetic mutations, and the causative genes are known to be parkin, PINK1, DJ-1, α-synuclein, and LRRK2 (leucine-rich repeat kinase 2).

Among these, the G2019S mutation in the LRRK2 gene is the most frequently occurring dominant genetic type, and LRRK2 is a protein belonging to the leucine-rich repeat kinase family, is composed of 2527 amino acid sequences with high interspecies similarity, and has both GTPase and serine-threonine kinase activities in one protein.

LRRK2 is reported to be highly expressed in dopaminergic neurons in the cerebral cortex, substantia nigra, striatum, hippocampus, and cerebellum, which are related to the pathogenesis of Parkinson's disease, and it is known that increased activity of LRRK2 affects the onset and progression of Parkinson's disease. It has been confirmed that when normal LRRK2 is overexpressed in neurons, apoptosis is induced, dopaminergic neuronal apoptosis, Lewy bodies, and motor disorders similar to Parkinson's disease symptoms have been reported in LRRK2 hyperactive mutant animals, and in the case of LRRK2 knock-out animals, inhibition of neurite outgrowth and age-dependent α-synuclein increase closely related to Parkinson's disease in the brain has been observed, so that the possibility of treating Parkinson's disease by LRRK2 inhibition has been confirmed in animal models. In addition, LRRK2 is known to be related to the transmission of mild cognitive impairment associated with Alzheimer's disease, and thus compounds and compositions effective in regulating LRRK2 activity may provide therapeutic effects for neurodegenerative diseases, etc. Therefore, selective inhibition of LRRK2 activity may be a useful target for the development of new drugs for the treatment of diseases.

In addition to the above neurodegenerative diseases, anticancer activity through LRRK2 inhibition is being studied in various cancer types as a mechanism for inhibiting cancer cell proliferation and promoting apoptosis. In particular, LRRK2 has been reported to be overexpressed in renal cancer, thyroid cancer, and liver cancer tissues, and it is known that the risk of breast cancer increases when LRRK2 is overactivated due to LRRK2 mutation occurrence (especially G2019S type). In addition, a study result was reported that patients with overexpressed LRRK2 in the tissues of domestic brain cancer patients had significantly shorter survival periods. In this regard, Boronoi recently received approval to enter phase 1 clinical trials for a LRRK2 inhibitor as a brain cancer treatment. Therefore, selective inhibition of LRRK2 activity may be a target for a wide range of new drug development, not only for the treatment of neurodegenerative diseases but also for anticancer treatment.

Meanwhile, in a prior art document related to a composition for the prevention or treatment of neurodegenerative diseases containing a pyrimidine derivative as an effective ingredient, pyrimidin-2-ylamino-1H-pyrazole was disclosed as an LRRK2 inhibitor for use in the treatment of neurodegenerative disorders (patent document, Korean Patent Publication No. 10-2019-0018676). However, there is no previous report that mentions a pyrimidine derivative such as Formula 1 of the present invention and the LRRK2 inhibitory activity of the derivative and the possibility of treating neurodegenerative diseases therefrom.

Accordingly, in a process of studying LRRK2 derivatives, the inventors of the present invention have completed the present invention by confirming that a macrocyclic pyrimidine derivative such as Formula 1 of the present invention has an excellent LRRK2 inhibitory activity effect and efficiently passes through the blood-brain barrier (BBB).

### DISCLOSURE OF THE INVENTION

### TECHNICAL PROBLEM

An aspect of the present invention is to provide a novel macrocyclic pyrimidine derivative.

Another aspect of the present invention is to provide a novel pharmaceutical composition for preventing or treating neurodegenerative disease.

Another aspect of the present invention is to provide a novel health functional food for preventing or alleviating neurodegenerative disease.

### TECHNICAL SOLUTION

To this end,
the present invention provides a compound represented by Formula 1, a solvate thereof, a hydrate thereof, or a pharmaceutically acceptable salt thereof. 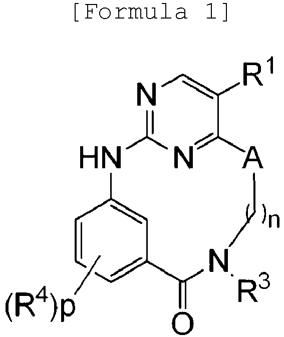
In Formula 1,
A is O or N(R²),
R¹ is hydrogen, halogen or C₁₋₁₀ alkyl which is unsubstituted or substituted with halogen;
R² and R³ are each independently hydrogen or unsubstituted C₁₋₁₀ alkyl;
p is 0 to 3;
R⁴ is independently selected from the group consisting of hydrogen, halogen, C₁₋₁₀ alkyl, or OR^{4a}, wherein R^{4a} is hydrogen, unsubstituted or halogen substituted C₁₋₁₀ alkyl, C₃₋₇ cycloalkyl, or 3-7 membered heterocycloalkyl; and n is 1 to 5.

In another aspect, the present invention provides a method for preparing a compound represented by Formula 1, comprising, as shown in Reaction Formula 1,
preparing a compound represented by Formula 1 by cyclizing a compound represented by Formula 1A. 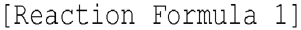 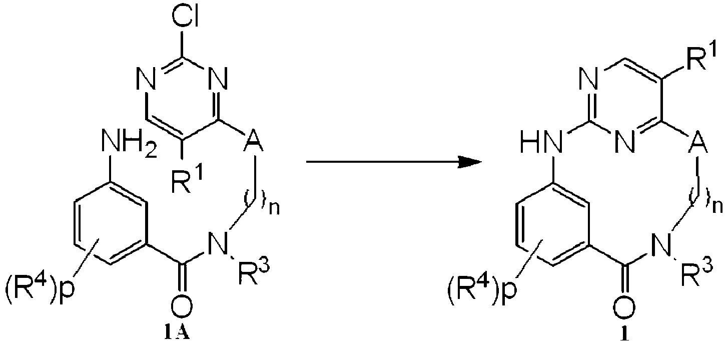
In Reaction Formula 1,
A, R¹, R³, R⁴, p and n are as defined in Formula 1.

In another aspect, the present invention provides a pharmaceutical composition for preventing or treating neurodegenerative disease, containing as an active ingredient a compound represented by Formula 1 described herein, a solvate thereof, a hydrate thereof, or a pharmaceutically acceptable salt thereof.

In another aspect, the present invention provides health functional food for preventing or alleviating neurodegenerative disease, containing, as an active ingredient, a compound represented by Formula 1 described herein, a solvate thereof, a hydrate thereof, or a pharmaceutically acceptable salt thereof.

### ADVANTAGEOUS EFFECTS

The macrocyclic pyrimidine derivative according to the present invention has excellent LRRK2 inhibitory activity and efficiently passes through the blood-brain barrier (BBB), so that the macrocyclic pyrimidine derivative may be usefully used as a pharmaceutical composition for preventing or treating neurodegenerative diseases.

### MODE FOR CARRYING OUT THE INVENTION

Hereinafter, the present invention will be described in detail.

Meanwhile, the embodiments of the present invention may be modified in various other forms, and the scope of the present invention is not limited to the embodiments described below. In addition, the embodiments of the present invention are provided to more completely explain the present invention to a person having average knowledge in the relevant technical field.

Furthermore, throughout the specification, the term "including" a certain component does not exclude other components unless specifically stated to the contrary, but means that other components may be further included.

The term "alkylene" or "alkyl" includes a straight or branched saturated hydrocarbon residue, unless otherwise specified. For example, "C₁₋₆ alkyl" means an alkyl having a skeleton of 1 to 6 carbons. Specifically, C₁₋₆ alkyl may include methyl, ethyl, n-propyl, i-propyl, n-butyl, i-butyl, t-butyl, n-pentyl, i-pentyl, t-pentyl, sec-pentyl, neopentyl, hexyl, etc. "Alkenyl" includes some unsaturated hydrocarbon residues containing one or more, ranging from 1 to 5, carbon-to-carbon double bonds within the "alkyl" hydrocarbon chain.

The term "cycloalkyl" includes a cyclized fully saturated hydrocarbon residue, and "3 to 7 membered cycloalkyl" means a cycloalkyl having 3-7 carbon atoms in the ring, such as cyclopropyl, cyclobutyl, cyclopentyl, cyclohexyl, and cycloheptyl. Unless otherwise specified, bridged, fused, or spiral cycloalkyls consisting of 2 or 3 rings may be included.

The term "heterocycloalkyl", unless otherwise specified, includes a monovalent saturated moiety consisting of 1 to 3 rings, which contain 1, 2, 3 or 4 heteroatoms selected from N, O or S as ring atoms forming a ring. In this case, the nitrogen (N) is included in the ring as NH to which hydrogen is bonded. The 2 or 3 rings may include bridged, fused or spiral heterocycloalkyl.

The term "heteroaryl", unless otherwise specified, may include an aromatic radical of a single ring or two or three fused rings having one or more aromatic rings containing, as ring atoms, one, two or three ring heteroatoms selected from N, O or S.

The term "substituted with halogen" means a state in which one or more halogen atoms are substituted, and the number of halogen atoms substituted is specifically in the range of 1 to 6, 1 to 5, or 1 to 4, for example, haloC₁₋₁₀alkyl means C₁₋₁₀alkyl in which carbon is substituted with 1 to 6 halogen atoms.

The term "alkylcarbonyl" or "alkenylcarbonyl" means alkyl or alkenyl substituted via a carbonyl group.

In this specification, unless otherwise indicated as "substituted", this case means unsubstituted.

An aspect of the present invention
provides a compound represented by Formula 1, a solvate thereof, a hydrate thereof, or a pharmaceutically acceptable salt thereof. 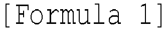 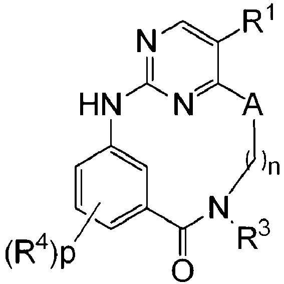
In Formula 1,
A is O or N(R²),
R¹ is hydrogen, halogen, or C₁₋₁₀ alkyl which is unsubstituted or substituted with halogen;
R² and R³ are each independently hydrogen or unsubstituted C₁₋₁₀ alkyl;
p is 0 to 3;
R⁴ is independently selected from the group consisting of halogen, C₁₋₁₀ alkyl, or OR^{4a}, wherein R^{4a} is hydrogen, unsubstituted or halogen substituted C₁₋₁₀ alkyl, C₃₋₇ cycloalkyl, or 3 to 7 membered heterocycloalkyl; and
n is 1 to 5.

In Formula 1 above, as another example,
R¹ is halogen, or C₁₋₆ alkyl which is unsubstituted or substituted with 1 to 5 halogens;
R² and R³ are each independently hydrogen, or C₁₋₆ alkyl;
p is 0 to 2;
R⁴ is independently selected from the group consisting of halogen, C₁₋₆ alkyl, or OR^{4a}, wherein R^{4a} is hydrogen, unsubstituted or halogen substituted C₁₋₆ alkyl, C₃₋₆ cycloalkyl, or 3 to 6 membered heterocycloalkyl; and
n is 1 to 5.

In Formula 1, as another example,
R¹ is halogen, or C₁₋₃ alkyl which is unsubstituted or substituted with 1 to 3 halogens;
R² and R³ are each independently hydrogen, or C₁₋₃ alkyl;
p is 0 to 2;
R⁴ is independently selected from the group consisting of halogen, C₁₋₃ alkyl, or OR^{4a}, wherein R^{4a} is hydrogen, unsubstituted or halogen substituted C₁₋₃ alkyl, C₃₋₆ cycloalkyl, or 3 to 6 membered heterocycloalkyl; and
n is 1 to 5.

In Formula 1 above, as another example,
R¹ is -Cl, -F, or CF₃;
R² and R³ are each independently hydrogen, or C₁₋₃ alkyl;
p is 0 to 2;
R⁴ is independently selected from the group consisting of -F, C₁₋₃ alkyl, or OR^{4a}, wherein R^{4a} is C₁₋₃ alkyl unsubstituted or substituted with 1 to 3 halogens, C₃₋₆ cycloalkyl, or 3-6 membered heterocycloalkyl containing one of O atom, and
n is 1 to 4.

In Formula 1, as another example,
R¹ is -Cl, -F, or -CF₃;
R² and R³ are each independently hydrogen, methylethyl or isopropyl;
p is 0 to 2;
R⁴ is independently -F, ethyl, -CF₃, methoxy, isopropoxy, -OCF₃, cyclopropoxy, oxetane-3-yloxy; and
n is 2 to 4.

In another example, as a compound according to Formula 1 above,
1⁵-bromo-2,5,10-triaza-1(2,4)-pyrimidina-3(1,3)-benzenacyclodecarphane-4-one; and
1⁵-bromo-2,5,11-triaza-1(2,4)-pyrimidina-3(1,3)-benzenecycloundecaphane-4-one are excluded.

As another example, a compound represented by Formula 1 is one selected from the following group of compounds.
<1> 3⁶-methoxy-5,9-dimethyl-1⁵-(trifluoromethyl)-2,5,9-triaza-1(2,4)-pyrimidina-3(1,3)-benzenacyclononaphan-4-one;2,2,2-trifluoroacetate;
<2> 1⁵-chloro-5-methyl-2,5,8-triaza-1(2,4)-pyrimidina-3(1,3)-benzenacyclooctaphane-4-one;
<3> 1⁵-chloro-2,5,10-triaza-1(2,4)-pyrimidina-3(1,3)-benzenacyclodecarphane-4-one;
<4> 1⁵-chloro-3⁶-methoxy-2,5,10-triaza-1(2,4)-pyrimidina-3(1,3)-benzenacyclodecarphane-4-one;
<5> 1⁵-chloro-3⁶-methoxy-2,5,8-triaza-1(2,4)-pyrimidina-3(1,3)-benzenacyclooctaphane-4-one;
<6> 1⁵-chloro-3⁶-methoxy-5-methyl-2,5,8-triaza-1(2,4)-pyrimidina-3(1,3)-benzenacyclooctaphane-4-one;2,2,2-trifluoroacetate;
<7> 1⁵-fluoro-3⁶-methoxy-5-methyl-2,5,8-triaza-1(2,4)-pyrimidina-3(1,3)-benzenacyclooctaphane-4-one;
<8> 1⁵-chloro-3⁶-fluoro-5-methyl-2,5,8-triaza-1(2,4)-pyrimidina-3(1,3)-benzenacyclooctaphane-4-one;2,2,2-trifluoroacetate;
<9> 1⁵-chloro-3⁴-methoxy-5-methyl-2,5,8-triaza-1(2,4)-pyrimidina-3(1,3)-benzenacyclooctaphane-4-one;2,2,2-trifluoroacetate;
<10> 1⁵-chloro-3⁵-methoxy-5-methyl-2,5,8-triaza-1(2,4)-pyrimidina-3(1,3)-benzenacyclooctaphane-4-one;2,2,2-trifluoroacetate;
<11> 1⁵-chloro-3⁶-methoxy-5-methyl-2,5,9-triaza-1(2,4)-pyrimidina-3(1,3)-benzenacyclononapane-4-one;2,2,2-trifluoroacetate;
<12> 1⁵-chloro-3⁶-methoxy-2,5,9-triaza-1(2,4)-pyrimidina-3(1,3)-benzenacyclononapane-4-one;2,2,2-trifluoroacetate;
<13> 1⁵-chloro-3⁶-ethyl-5-methyl-2,5,9-triaza-1(2,4)-pyrimidina-3(1,3)-benzenacyclononapane-4-one;2,2,2-trifluoroacetate;
<14> 1⁵-chloro-3⁵-methoxy-5-methyl-2,5,9-triaza-1(2,4)-pyrimidina-3(1,3)-benzenacyclononapane-4-one;2,2,2-trifluoroacetate;
<15> 1⁵-chloro-3⁴-methoxy-5-methyl-2,5,9-triaza-1(2,4)-pyrimidina-3(1,3)-benzenacyclononapane-4-one;2,2,2-trifluoroacetate;
<16> 1⁵-chloro-3⁶-methoxy-5-methyl-2,5,10-triaza-1(2,4)-pyrimidina-3(1,3)-benzenacyclodecarphane-4-one;2,2,2-trifluoroacetate;
<17> 1⁵-chloro-3⁵-methoxy-5-methyl-2,5,10-triaza-1(2,4)-pyrimidina-3(1,3)-benzenacyclodecarphane-4-one;2,2,2-trifluoroacetate;
<18> 1⁵-chloro-3⁴-methoxy-5-methyl-2,5,10-triaza-1(2,4)-pyrimidina-3(1,3)-benzenacyclodecarphane-4-one;2,2,2-trifluoroacetate;
<19> 1⁵-chloro-3⁶-ethyl-5-methyl-2,5,10-triaza-1(2,4)-pyrimidina-3(1,3)-benzenacyclodecarphane-4-one;2,2,2-trifluoroacetate;
<20> 1⁵-chloro-3⁶-methoxy-5,8-dimethyl-2,5,8-triaza-1(2,4)-pyrimidina-3(1,3)-benzenacyclooctaphane-4-one;2,2,2-trifluoroacetate;
<21> 1⁵-chloro-5-ethyl-3⁶-methoxy-2,5,8-triaza-1(2,4)-pyrimidina-3(1,3)-benzenacyclooctaphane-4-one;2,2,2-trifluoroacetate;
<22> 1⁵-chloro-5-isopropyl-3⁶-methoxy-2,5,8-triaza-1(2,4)-pyrimidina-3(1,3)-benzenacyclooctaphane-4-one;
<23> 1⁵-chloro-3⁶-methoxy-5,9-dimethyl-2,5,9-triaza-1(2,4)-pyrimidina-3(1,3)-benzenacyclononapane-4-one;2,2,2-trifluoroacetate;
<24> 3⁶-methoxy-5-methyl-1⁵-(trifluoromethyl)-2,5,9-triaza-1(2,4)-pyrimidina-3(1,3)-benzenacyclononapane-4-one;2,2,2- trifluoroacetate;
<25> 3⁶-methoxy-5,8-dimethyl-1⁵-(trifluoromethyl)-2,5,8-triaza-1(2,4)-pyrimidina-3(1,3)-benzenacyclooctaphane-4-one;2,2, 2-trifluoroacetate;
<26> 3⁶-methoxy-5-methyl-1⁵-(trifluoromethyl)-2,5,8-triaza-1(2,4)-pyrimidina-3(1,3)-benzenacyclooctaphane-4-one;2,2,2- trifluoroacetate;
<27> 1⁵-chloro-3⁶-methoxy-5,10-dimethyl-2,5,10-triaza-1(2,4)-pyrimidina-3(1,3)-benzenacyclodecarphane-4-one;2,2,2-trifluoroacetate;
<28> 3⁵-fluoro-3⁶-methoxy-5,9-dimethyl-1⁵-(trifluoromethyl)-2,5,9-triaza-1(2,4)-pyrimidina-3(1,3)-benzenacyclononapane-4-one;
<29> 3⁶-methoxy-5,10-dimethyl-1⁵-(trifluoromethyl)-2,5,10-triaza-1(2,4)-pyrimidina-3(1,3)-benzenacyclodecarphane-4-one;2,2, 2-trifluoroacetate;
<30> 5,9-dimethyl-3⁶-(trifluoromethoxy)-1⁵-(trifluoromethyl)-2,5,9-triaza-1(2,4)-pyrimidina-3(1,3)-benzenacyclononapane-4-one;
<31> 5,9-dimethyl-1⁵,3⁶-bis(trifluoromethyl)-2,5,9-triaza-1(2,4)-pyrimidina-3(1,3)-benzenacyclononapane-4-one;
<32> 3⁵-methoxy-5,9-dimethyl-1⁵-(trifluoromethyl)-2,5,9-triaza-1(2,4)-pyrimidina-3(1,3)-benzenacyclononaphan-4-one;2,2,2-trifluoroacetate;
<33> 3⁶-methoxy-5-methyl-1⁵-(trifluoromethyl)-2,5,10-triaza-1(2,4)-pyrimidina-3(1,3)-benzenacyclodecarphane-4-one;2,2,2-trifluoroacetate;
<34> 3⁵-fluoro-3⁶-methoxy-5-methyl-1⁵-(trifluoromethyl)-2,5,10-triaza-1(2,4)-pyrimidina-3(1,3)-benzenacyclodecarphane-4-one;
<35> 5-methyl-3⁶-(trifluoromethoxy)-1⁵-(trifluoromethyl)-2,5,10-triaza-1(2,4)-pyrimidina-3(1,3)-benzenacyclodecarphane-4-one;
<36> 3⁵-fluoro-3⁶-methoxy-5-methyl-1⁵-(trifluoromethyl)-2,5,9-triaza-1(2,4)-pyrimidina-3(1,3)-benzenacyclonaphane-4-one;
<37> 3⁵-methoxy-5-methyl-1⁵-(trifluoromethyl)-2,5,9-triaza-1(2,4)-pyrimidina-3(1,3)-benzenacyclonaphane-4-one;
<38> 5-methyl-3⁶-(oxetane-3-yloxy)-1⁵-(trifluoromethyl)-2,5,9-triaza-1(2,4)-pyrimidina-3(1,3)-benzenacyclonaphane-4-one;
<39> 3⁶-isopropoxy-5-methyl-1⁵-(trifluoromethyl)-2,5,9-triaza-1(2,4)-pyrimidina-3(1,3)-benzenacyclonaphane-4-one;
<40> 1⁵-chloro-5-methyl-3⁶-(trifluoromethoxy)-2,5,9-triaza-1(2,4)-pyrimidina-3(1,3)- benzenacyclonaphane-4-one;
<41> 5-methyl-3⁶-(trifluoromethoxy)-1⁵-(trifluoromethyl)-2,5,9-triaza-1(2,4)-pyrimidina-3(1,3)-benzenacyclonaphane-4-one;
<42> 15-chloro-36-methoxy-5-methyl-9-oxa-2,5-diaza-1(2,4)-pyrimidina-3(1,3)-benzenacyclonaphane-4-one;
<43> 3⁶-methoxy-5-methyl-1⁵-(trifluoromethyl)-9-oxa-2,5-diaza-1(2,4)-pyrimidina-3(1,3)-benzenacyclonaphane-4-one;
<44> 5-methyl-3⁶-(trifluoromethoxy)-1⁵-(trifluoromethyl)-9-oxa-2,5-diaza-1(2,4)-pyrimidina-3(1,3)-benzenacyclonaphane-4-one;
<45> 3⁵-fluoro-3⁶-methoxy-5-methyl-1⁵-(trifluoromethyl)-9-oxa-2,5-diaza-1(2,4)-pyrimidina-3(1,3)-benzenacyclonaphane-4-one;
<46> 3⁶-cyclopropoxy-5-methyl-1⁵-(trifluoromethyl)-2,5,9-triaza-1(2,4)-pyrimidina-3(1,3)-benzenacyclonaphane-4-one; and
<47> 3⁶-cyclopropoxy-5,9-dimethyl-1⁵-(trifluoromethyl)-2,5,9-triaza-1(2,4)-pyrimidina-3(1,3)-benzenacyclonaphane-4-one.

The compound represented by the Formula 1 of the present invention may be used in the form of a pharmaceutically acceptable salt, and as a salt, an acid addition salt formed by a pharmaceutically acceptable free acid is useful. The acid addition salt is obtained from inorganic acids such as hydrochloric acid, nitric acid, phosphoric acid, sulfuric acid, hydrobromic acid, hydroiodic acid, nitrous acid, and phosphorous acid, non-toxic organic acids such as aliphatic mono- and dicarboxylates, phenyl-substituted alkanoates, hydroxy alkanoates and alkanedioates, aromatic acids, and aliphatic and aromatic sulfonic acids, organic acids such as trifluoroacetate, acetate, benzoic acid, citric acid, lactic acid, maleic acid, gluconic acid, methanesulfonic acid, 4-toluenesulfonic acid, tartaric acid, and fumaric acid. These pharmaceutically nontoxic salts include sulfates, pyrosulfates, bisulfates, sulfites, bisulfites, nitrates, phosphates, monohydrogen phosphates, dihydrogen phosphates, metaphosphates, pyrophosphate chlorides, bromides, iodides, fluorides, acetates, propionates, decanoates, caprylates, acrylates, formates, isobutyrates, caprates, heptanoates, propiolates, oxalates, malonates, succinates, suberates, sebacates, fumarates, maleates, butyne-1,4-dioate, hexane-1,6-dioate, benzoates, chlorobenzoates, methylbenzoates, dinitrobenzoates, hydroxybenzoates, methoxybenzoates, phthalates, terephthalate, benzene sulfonate, toluene sulfonate, chlorobenzene sulfonate, xylene sulfonate, phenyl acetate, phenyl propionate, phenyl butyrate, citrate, lactate, β-hydroxybutyrate, glycolate, malate, tartrate, methane sulfonate, propane sulfonate, naphthalene-1-sulfonate, naphthalene-2-sulfonate, mandelate, etc.

The acid addition salt according to the present invention may be prepared by a general method, for example, by dissolving a derivative of Formula 1 in an organic solvent such as methanol, ethanol, acetone, methylene chloride, and acetonitrile, adding an organic acid or an inorganic acid, filtering and drying the resulting precipitate, or by distilling the solvent and an excess of acid under reduced pressure, drying, and crystallizing in the organic solvent.

The term "hydrate" means a compound of the present invention or a salt thereof containing a stoichiometric or non-stoichiometric amount of water bound by non-covalent intermolecular force. The hydrate of the compound represented by Formula 1 of the present invention may contain a stoichiometric or non-stoichiometric amount of water bound by non-covalent intermolecular force. The hydrate may contain 1 equivalent or more, preferably 1 to 5 equivalents of water. Such hydrates may be prepared by crystallizing a compound represented by Formula 1 of the present invention, an isomer thereof, or a pharmaceutically acceptable salt thereof from water or a solvent containing water.

The term "solvate" means a compound of the present invention or a salt thereof containing a stoichiometric or non-stoichiometric amount of a solvent bound by non-covalent intermolecular forces. Preferred solvents therefor include solvents that are volatile, non-toxic, and/or suitable for administration to humans.

In another aspect of the present invention,
as shown in Reaction Formula 1 below,
a method for preparing a compound represented by Formula 1, which includes (step 4) preparing a compound represented by Formula 1 by cyclizing a compound represented by Formula 1A,
is provided.

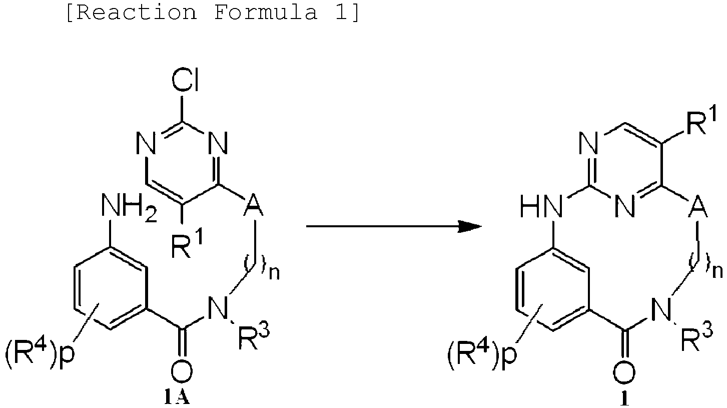

In Reaction Formula 1,
A, R¹, R³, R⁴ , p and n are as defined in Formula 1.

This is a step of reacting the compound represented by Formula 1A in Reaction Formula 1 under a metal catalyst to cause a cyclization reaction. The cyclization reaction is achieved through a substitution reaction of the chloro substituent of the pyrimidine ring by an amino group.

For this reaction, the metal catalyst is typically palladium, and tetrakistriphenylphosphine palladium(0) ((PPh₃)₄Pd), palladium acetate (IItris (dibenzylideneacetone)dipalladium(0) (Pd₂dba₃), bis(triphenylphosphine)palladium(II chloride(PdCl₂(PPh₃)₂), and [1,1'-bis(diphenylphosphino)ferrocene]palladium(0) chloride(Pd(dppf)Cl₂) may be used. This reaction may be carried out without using a ligand, but the reaction is generally carried out using ligands such as triphenylphosphine ((PPh₃)₄), 1,2-bis(diphenylphosphino)propane (DPPP), 2,2'-bis(diphenylphosphino)-1,1'-binaphthyl (BINAP), Xantphos, and XPhos, which are commonly used in the Buchwald reaction under a metal catalyst. Bases that may be used at this time include potassium carbonate, KOtBu, cesium carbonate, potassium phosphate, sodium hydroxide, triethylamine, etc., and the reaction is performed using solvents that do not adversely affect the reaction, such as toluene, tetrahydrofuran, dioxane, and N,N-dimethylformamide. The reaction conditions for the cyclization reaction may be carried out under acidic conditions or basic conditions, and may be applied as known in the art.

After step 4, a reaction for forming a salt form may be further included. The reaction conditions for forming the salt may utilize the organic acid or inorganic acid defined above, and may be applied as known in the art.

Formula 1A of Reaction Formula 1 may be prepared as Reaction Formula 2.

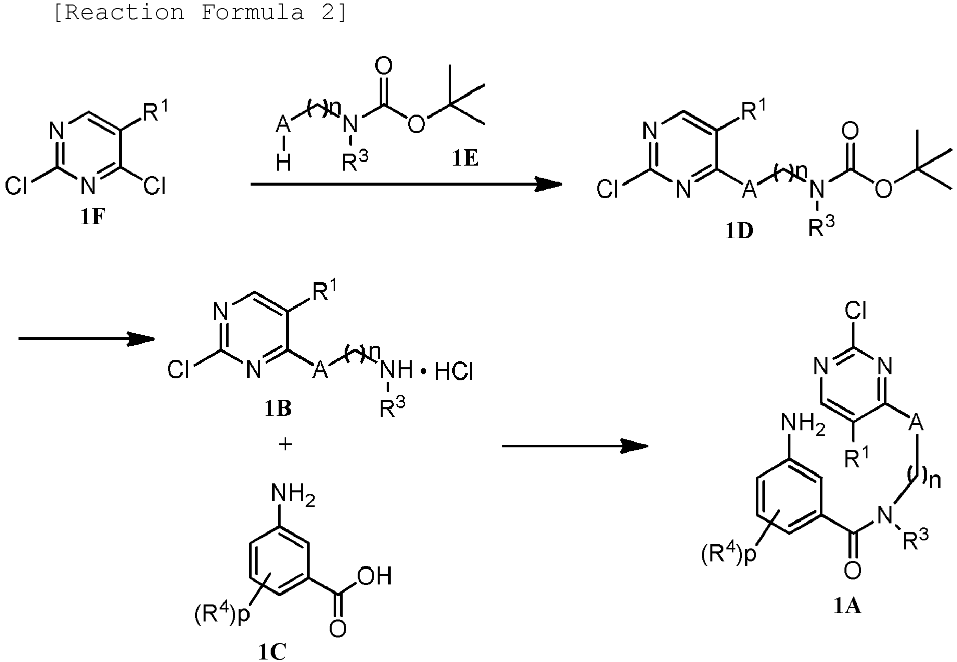

In Reaction Formula 2,
A, R¹, R³, R⁴, p and n are as defined in Formula 1.
B is hydrogen.

Hereinafter, a production method of Reaction Formula 2 is described in detail.

Step 1 of Reaction Formula 2 is preparing a compound represented by Formula 1C by reacting a compound represented by Formula 1F with 1E through a Burkwald-Hartwick amination reaction (when A is N-R²) or a substitution reaction by an alkoxide (when A is O). The reaction is preferably performed in the presence of a palladium catalyst and a base, and the reactor for the Burkwald-Hartwick amination may be changed according to what is known in the art. A strong base such as a hydride may be required for the substitution reaction by an alkoxide.

Step 2 is a hydrolysis reaction, which is a reaction that changes a carbamate into a secondary amine form under acid conditions. The reaction is preferably performed under acid conditions, and the reaction conditions for the hydrolysis reaction may be applied according to what is known in the art.

Step 3 is preparing Formula 1A by reacting Formula 1B with various benzoic acids 1C as an amidation reaction. The reaction conditions for the above amidation reaction may be applied according to what is known in the art.

The production method is not limited to an embodiment of the present invention presented as an example, and may be performed by modifying a solvent, a reactant, temperature conditions, etc. under general organic chemical knowledge.

In another aspect of the present invention,
provided is a pharmaceutical composition for preventing or treating neurodegenerative disease, containing, as an active ingredient, a compound represented by Formula 1 above, a solvate thereof, a hydrate thereof, or a pharmaceutically acceptable salt thereof.

The neurodegenerative disease may be Parkinson's disease, Alzheimer's disease, Huntington's disease, Pick's disease, Amyotrophic lateral sclerosis, Prion disease, Motor neuron disease, Spinocerebellar ataxia, Spinal muscular atrophy, Creutzfeldt-Jakob disease, or Alcohol related dementia.

The compound may inhibit LRRK2 (leucine-rich repeat kinase 2) activity.

In the present invention, the term "containing as an effective ingredient" means containing as an ingredient that plays a role in preventing, alleviating, or treating neurodegenerative disease, and at this time, the dosage range may vary depending on the severity and formulation, and the number of applications may vary depending on the age, weight, and constitution of the subject of application. In a specific example of the present invention, the compound represented by Formula 1 in the pharmaceutical composition of the present invention is included in, for example, 0.001 mg/kg or more, 0.1 mg/kg or more, 10 mg/kg or more, 100 mg/kg or more, 250 mg/kg or more, or 0.1 g/kg or more. The quantitative upper limit of the compound represented by Formula 1 included in the pharmaceutical composition of the present invention may be selected and implemented within an appropriate range by a person skilled in the art. This may be selected within a range that may have activity without exhibiting fatal toxicity, and for example, this may be 2000 mg/kg or less, 1000 mg/kg or less.

The pharmaceutical composition according to the present invention may contain an effective amount of a compound represented by Formula 1 alone or may contain one or more pharmaceutically acceptable carriers, excipients or diluents.

The pharmaceutically acceptable carriers, excipients or diluents refer to a substance that is physiologically acceptable and does not usually cause allergic reactions such as gastrointestinal upset, dizziness or similar reactions when administered to humans. Examples of the above carriers, excipients and diluents include, but are not limited to, lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate and mineral oil. In addition, fillers, anticoagulants, lubricants, wetting agents, fragrances, emulsifiers and preservatives may be additionally included.

The compound represented by Formula 1 above or a pharmaceutically acceptable salt thereof may be administered in various oral and parenteral dosage forms during clinical administration. When formulated, this is prepared using diluents or excipients such as fillers, bulking agents, binders, wetting agents, disintegrants, and surfactants that are commonly used. Solid preparations for oral administration include tablets, pills, powders, granules, capsules, etc., and these solid preparations are prepared by mixing one or more compounds with at least one excipient, such as starch, calcium carbonate, sucrose, lactose, or gelatin. In addition to simple excipients, lubricants such as magnesium stearate and talc are also used. Liquid preparations for oral administration include suspensions, solutions, emulsions, and syrups, and in addition to the commonly used simple diluents such as water and liquid paraffin, liquid preparations may include various excipients such as wetting agents, sweeteners, fragrances, and preservatives. Preparations for parenteral administration include sterile aqueous solutions, non-aqueous solvents, suspensions, and emulsions. Non-aqueous solvents and suspensions include propylene glycol, polyethylene glycol, vegetable oils such as olive oil, and injectable esters such as ethyl oleate.

The pharmaceutical composition containing the compound represented by Formula 1 above or a pharmaceutically acceptable salt thereof as an active ingredient may be administered parenterally, and parenteral administration is by subcutaneous injection, intravenous injection, intramuscular injection, or intrathoracic injection.

In this case, in order to formulate a formulation for parenteral administration, the compound represented by Formula 1 above or a pharmaceutically acceptable salt thereof is mixed with a stabilizer or buffer in water to prepare a solution or suspension, which may be prepared in an ampoule or vial unit dosage form. The composition may be sterilized and/or contain auxiliary agents such as preservatives, stabilizers, hydrating agents or emulsifying promoters, salts for osmotic pressure control, and/or buffers, and other therapeutically useful substances, and may be formulated by conventional mixing, granulation, or coating methods.

In the present invention, the term "prevention" means that the pharmaceutical composition or food composition of the present invention is administered, ingested, or applied to a subject not suffering from neurodegenerative disease, thereby suppressing or blocking the symptoms of neurodegenerative disease, thereby preventing the occurrence of cancer symptoms in advance.

In the present invention, the term "treatment" means that the pharmaceutical composition of the present invention is administered to a subject suffering from a neurodegenerative disease, and includes complete cure of the symptoms of a neurodegenerative disease as well as partial cure, mitigation, and alleviation of the symptoms of neurodegenerative disease.

The pharmaceutical composition of the present invention is administered in a pharmaceutically effective amount.

In the present invention, the term "pharmaceutically effective amount" means an amount sufficient to treat a disease with a reasonable benefit/risk ratio applicable to medical treatment or alleviation, and the effective dosage level may be determined according to factors including the type and severity of the individual, age, sex, activity of the drug, sensitivity to the drug, administration time, administration route and excretion rate, treatment period, concurrently used drugs, and other factors well known in the medical field.

Furthermore, the compound represented by Formula 1 above may be used in the form of not only a pharmaceutically acceptable salt thereof, but also a solvate, hydrate, etc. that may be prepared therefrom.

In another aspect of the present invention,
provided is a health functional food for preventing or alleviating neurodegenerative disease, containing containing as an active ingredient a compound represented by Formula 1, a solvate thereof, a hydrate thereof, or a pharmaceutically acceptable salt thereof.

In the present invention, the term "alleivation" means including mitigation or relief of symptoms of neurodegenerative disease by administering, ingesting, or applying the pharmaceutical composition or food composition of the present invention to a subject suffering from neurodegenerative disease.

In the present invention, the term "health functional food" may be used interchangeably with "health supplementary food" and means a food produced and processed using raw materials or ingredients having useful functionality for the human body according to the Health Functional Food Act, and the term "functionality" means ingestion for the purpose of obtaining a useful effect for health purposes such as regulating nutrients for the structure and function of the human body or physiological effects. The health functional food or health supplementary food of the present invention obtained in this way is very useful because this may be consumed on a daily basis. In the case of health functional foods or health supplements in the form of pills, granules, tablets or capsules, this is usually added in the range of 0.1 to 100 wt%, preferably 0.5 to 80 wt%. In a specific example, the health functional foods or health supplements of the present invention may be in the form of pills, tablets, capsules or beverages.

The matters mentioned in the pharmaceutical composition and food composition of the present invention are applied equally unless they are contradictory.

In another aspect, the present invention provides a method for preventing or treating a neurodegenerative disease, comprising administering to a subject in need thereof a compound represented by Formula 1 described herein, a solvate thereof, a hydrate thereof, or a pharmaceutically acceptable salt thereof.

In another aspect, the present invention provides a use of a compound represented by Formula 1 described herein, a solvate thereof, a hydrate thereof, or a pharmaceutically acceptable salt thereof, in preventing or treating a neurodegenerative disease.

In the method or use, the detailed description of the pharmaceutical composition or health functional food described above may be applied.

Hereinafter, the present invention will be described in detail through examples and experimental examples.

However, the examples and experimental examples described below are only specific examples of the present invention in an aspect, and the present invention is not limited thereto.

### <Example 1> 3⁶-methoxy-5-methyl-1⁵-(trifluoromethyl)-2,5,9-triaza-1(2,4)-pyrimidina-3(1,3)-benzenacyclononapane-4-one;2,2,2- trifluoroacetateproduction

### Step 1:

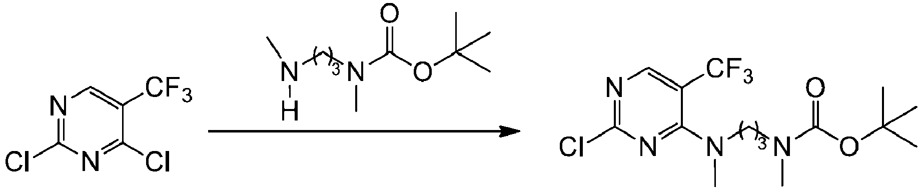

To a stirred solution obtained by dissolving 2,4-dichloro-5-(trifluoromethyl)pyrimidine (1.0 equivalent) in anhydrous tetrahydrofuran, N-tert-butoxycarbonyl-N,N'-dimethyl-1,3-propanediamine (1.2 eq) and triethylamine (1.2 eq) were added at 0°C. The resulting solution was warmed to room temperature and stirred for 1 hour. After completion of the reaction, the reaction mixture was concentrated under reduced pressure, and the mixture was extracted with dichloromethane and washed with brine. The organic layer was dried over anhydrous sodium sulfate, filtered, concentrated under reduced pressure, and purified by column chromatography to obtain the desired product.

### Step 2:

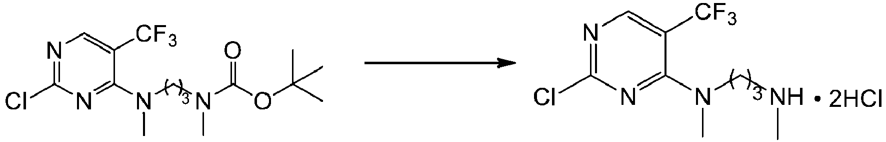

2-chloro-4-alkyl amine-substituted pyrimidine containing Boc- was added to 6.0 M hydrogen chloride and stirred at 40°C for 1 hour. After that, the solvent was depressurized to obtain the desired amine salt, which was used directly in the next reaction.

### Step 3:

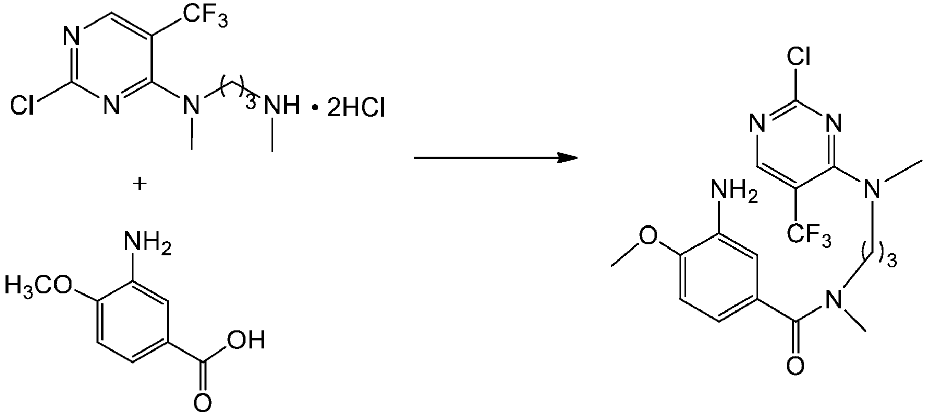

Pyrimidine salt (1.0 equivalent) was dissolved in anhydrous dimethylformamide solvent, 3-amino-benzoic acid (1.0 equivalent), 1-[Bis(dimethylamino)methylene]-1H-1,2,3-triazolo[4,5-b]pyridinium 3-oxide hexafluorophosphate (1-[Bis(dimethylamino)methylene]-1H-1,2,3-triazolo[4,5-b]pyridinium 3-oxide hexafluorophosphate; Hexafluorophosphate Azabenzotriazole Tetramethyl Uronium; HATU) (1.2 equivalent), and triethylamine (5.2 equivalent) were added, and then stirred at room temperature for 1 hour. After the reaction was completed, the mixture was diluted by adding water and ethyl acetate in an ice bath, and then washed with brine. The organic layer was dried over anhydrous sodium sulfate and filtered, and the solvent in the mixture was concentrated under reduced pressure, and then purified through column chromatography to obtain the desired product.

### Step 4:

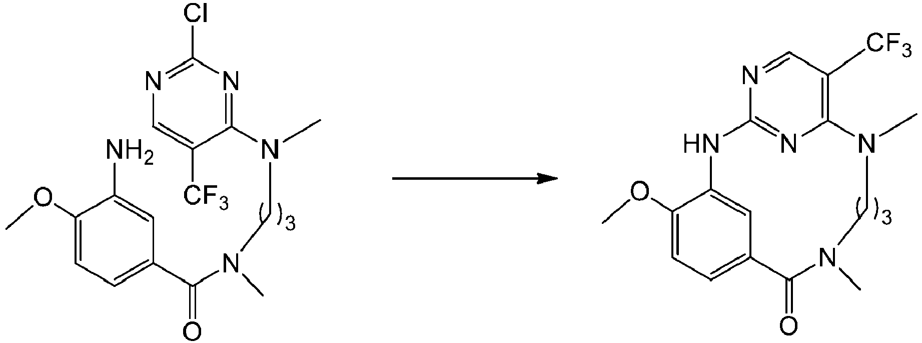

According to the reaction formula, 3-amino-N-(3-((2-chloro-5-(trifluoromethyl)pyrimidin-4-yl)(methyl)amino)propyl)-4-methoxy-N-methylbenzamide (86 mg) was dissolved in 1,4-dioxane (4.0 mL), and then palladium(II) acetate (9.0 mg), xanthos (46.2 mg), and tribasic potassium phosphate (84.7 mg) were added. The mixture was bubbled through the mixture under nitrogen for 5 minutes. Then, the mixture was heated to 90°C and stirred for 16 hours. After the reaction was completed, the mixture was diluted with ethyl acetate, the solid was removed through a Celite filter, and the mixture was washed with brine (20 mL). The organic layer was dried over anhydrous sodium sulfate, filtered, and the solvent of the mixture was concentrated under reduced pressure and purified through column chromatography to obtain 3⁶-methoxy-5,9-dimethyl-1⁵-(trifluoromethyl)-2,5,9-triaza-1(2,4)-pyrimidina-3(1,3)-benzenacyclonaphane-4-one (light yellow oil, 57 mg, 72%).

### Step 5:

### 3⁶-methoxy-5-methyl-1⁵-(trifluoromethyl)-2,5,9-triaza-1(2,4)-pyrimidina-3(1,3)-benzenacyclononapane-4-one;2,2,2-trifluoroacetate production

To prepare a salt form, the compound (1.0 equivalent) was dissolved in dichloromethane, a trifluoroacetate solution (1.5 equivalent) was added, and then the mixture was stirred at room temperature for 10-30 minutes. The solvent and excess trifluoroacetate were concentrated under reduced pressure and slowly evaporated to obtain the desired salt form of the compound.

¹H NMR (400 MHz, CD₂Cl₂) δ11.90 (bs, 1H), 10.84 (s, 1H), 8.28 (s, 1H), 7.87 (d, J = 2.1 Hz, 1H), 7.38 (dd, J = 8.5, 2.1 Hz, 1H), 7.05 (d, J = 8.5 Hz, 1H), 3.93 (s, 3H), 3.35 - 3.30 (m, 3H), 3.23 (bs, 4H), 3.06 (s, 3H), 2.20 (bs, 2H).

Hereinafter, compounds of Example 2 were prepared similarly to Example 1. The examples obtained in the form of TFA salts were performed up to step 5, and the non-salt forms were performed only up to step 4.

### <Example 2> 1⁵-chloro-5-methyl-2,5,8-triaza-1(2,4)-pyrimidina-3(1,3)-benzenacyclooctaphane-4-one production

¹H NMR (400 MHz, DMSO-d₆) δ9.28 (d, J = 3.6 Hz, 1H), 8.73 (s, 1H), 7.95 (d, J = 3.7 Hz, 1H), 7.52 (s, 1H), 7.25 (td, J = 7.5, 3.6 Hz, 1H), 7.21 (s, 1H), 7.05 (d, J = 4.9 Hz, 1H), 4.28 (t, *J* = 12.5 Hz, 1H), 3.76 - 3.64 (m, 1H), 3.53 (t, *J* = 12.9 Hz, 1H), 2.97 (d, *J* = 3.6 Hz, 3H), 2.91 - 2.81 (m, 1H).

### <Example 3> 1⁵-chloro-2,5,10-triaza-1(2,4)-pyrimidina-3(1,3)-benzenacyclodecarphane-4-one (1⁵-chloro-2,5,10-triaza-1(2,4)-pyrimidina-3(1,3)-benzenacyclodecaphan-4-one) production

¹H NMR (400 MHz, DMSO-d₆) δ9.52 (s, 1H), 9.32 (s, 1H), 8.28 (s, 1H), 7.95 (s, 1H), 7.45 (t, *J* = 5.9 Hz, 1H), 7.27 (t, *J* = 7.8 Hz, 1H), 7.14 (d, *J* = 8.1 Hz, 1H), 7.07 (d, *J* = 7.5 Hz, 1H), 3.26 (dt, *J* = 11.6, 6.2 Hz, 2H), 3.18 (q, *J* = 6.0 Hz, 2H), 1.79 (bs, 2H), 1.41 (bs, 2H).

### <Example 4> 1⁵-chloro-3⁶-methoxy-2,5,10-triaza-1(2,4)-pyrimidina-3(1,3)-benzenacyclodecarphane-4-one production

¹H NMR (400 MHz, DMSO-d₆) δ9.25 (bs, 1H), 8.15 (bs, 1H), 7.95 (s, 1H), 7.72 (s, 1H), 7.51 (t, *J* = 6.1 Hz, 1H), 7.18 (dd, *J* = 8.3, 2.1 Hz, 1H), 7.04 (d, *J* = 8.4 Hz, 1H), 3.89 (s, 3H), 3.31 - 3.22 (m, 2H), 3.17 (dt, *J* = 6.4, 4.6 Hz, 2H), 1.85 - 1.71 (m, 2H), 1.43 - 1.33 (m, 2H).

### <Example 5> 1⁵-chloro-3⁶-methoxy-2,5,8-triaza-1(2,4)-pyrimidina-3(1,3)-benzenacyclooctaphane-4-one production

¹H NMR (400 MHz, CD₂Cl₂) δ10.50 (s, 1H), 10.10 (bs, 1H), 8.89 (d, *J* = 2.1 Hz, 1H), 7.91 (s, 1H), 7.44 (dd, *J* = 8.5, 2.1 Hz, 1H), 6.99 (d, *J* = 8.5 Hz, 1H), 6.86 - 6.74 (m, 1H), 6.58 - 6.46 (m, 1H), 4.21 (q, *J* = 11.3 Hz, 1H), 3.93 (s, 3H), 3.82 - 3.64 (m, 2H), 3.30 - 3.10 (m, 1H).

### <Example 6> 1⁵-chloro-3⁶-methoxy-5-methyl-2,5,8-triaza-1(2,4)-pyrimidina-3(1,3)-benzenacyclooctaphane-4-one;2,2,2-trifluoroacetate production

¹H NMR (400 MHz, CD₂Cl₂) δ10.76 (bs, 1H), 10.39 (s, 1H), 8.64 (d, *J* = 2.2 Hz, 1H), 7.89 (s, 1H), 7.38 (dd, *J* = 8.5, 2.1 Hz, 1H), 6.95 (d, *J* = 8.5 Hz, 1H), 6.61 (s, 1H), 4.53 - 4.38 (m, 1H), 4.01 - 3.87 (m, 1H), 3.90 (s, 3H), 3.76 (ddt, J = 13.5, 10.8, 3.1 Hz, 1H), 3.07 (s, 3H), 3.00 (ddd, J = 13.7, 11.0, 6.9 Hz, 1H).

### <Example 7> 1⁵-fluoro-3⁶-methoxy-5-methyl-2,5,8-triaza-1(2,4)-pyrimidina-3(1,3)-benzenacyclooctaphane-4-one production

¹H NMR (400 MHz, DMSO) δ8.74 (d, *J* = 2.2 Hz, 1H), 7.90 (d, *J* = 4.5 Hz, 1H), 7.84 (dd, *J* = 6.2, 3.5 Hz, 1H), 7.79 (s, 1H), 7.11 (dd, *J* = 8.3, 2.1 Hz, 1H), 6.98 (d, *J* = 8.4 Hz, 1H), 4.28 - 4.16 (m, 1H), 3.85 (s, 3H), 3.67 (tt, *J* = 12.5, 6.5 Hz, 1H), 3.52 (tt, *J* = 13.2, 2.9 Hz, 1H), 2.95 (s, 3H), 2.83 (ddd, *J* = 13.2, 10.7, 6.9 Hz, 1H).

### <Example 8> 1⁵-chloro-3⁶-fluoro-5-methyl-2,5,8-triaza-1(2,4)-pyrimidina-3(1,3)-benzenacyclooctaphane-4-one;2,2,2-trifluoroacetate production

¹H NMR (400 MHz, CD₂Cl₂) δ11.07 (s, 1H), 8.93 (bs, 1H), 8.67 (dd, *J* = 7.2, 2.2 Hz, 1H), 8.00 (s, 1H), 7.41 (ddd, *J* = 8.5, 4.5, 2.2 Hz, 1H), 7.20 (dd, *J* = 10.3, 8.5 Hz, 1H), 6.74 (t, *J* = 5.2 Hz, 1H), 4.46 - 4.31 (m, 1H), 3.97 (tt, *J* = 12.8, 6.6 Hz, 1H), 3.82 - 3.67 (m, 1H), 3.13 (s, 3H), 3.12 - 3.04 (m, 1H).

### <Example 9> 1⁵-chloro-3⁴-methoxy-5-methyl-2,5,8-triaza-1(2,4)-pyrimidina-3(1,3)-benzenacyclooctaphane-4-one;2,2,2-trifluoroacetate production

¹H NMR (400 MHz, CD₂Cl₂) δ11.36 (s, 1H), 8.19 (d, *J* = 2.6 Hz, 1H), 7.86 (s, 1H), 7.25 (dd, *J* = 8.9, 2.7 Hz, 1H), 6.90 (d, *J* = 8.9 Hz, 1H), 6.52 (s, 1H), 6.16 (bs, 1H), 4.25 (t, *J* = 12.4 Hz, 1H), 3.88 (tt, *J* = 12.6, 6.6 Hz, 1H), 3.79 (s, 3H), 3.64 (t, *J* = 12.6 Hz, 1H), 3.14 (s, 3H), 3.12 - 3.01 (m, 1H).

### <Example 10> 1⁵-chloro-3⁵-methoxy-5-methyl-2,5,8-triaza-1(2,4)-pyrimidina-3(1,3)-benzenacyclooctaphane-4-one;2,2,2-trifluoroacetate production

¹H NMR (400 MHz, CD₂Cl₂) δ11.54 (s, 1H), 8.15 (s, 1H), 7.88 (s, 1H), 6.91 (dd, *J* = 2.4, 1.4 Hz, 1H), 6.86 (t, *J* = 2.1 Hz, 1H), 6.56 (s, 1H), 6.15 (bs, 1H), 4.44 (t, *J* = 12.5 Hz, 1H), 3.91 (tt, *J* = 12.6, 6.6 Hz, 1H), 3.80 (s, 3H), 3.75 (ddd, *J* = 14.0, 8.6, 3.4 Hz, 1H), 3.10 (s, 3H), 3.02 (ddd, *J* = 13.5, 10.9, 6.9 Hz, 1H).

### <Example 11> 1⁵-chloro-3⁶-methoxy-5-methyl-2,5,9-triaza-1(2,4)-pyrimidina-3(1,3)-benzenacyclononapane-4-one;2,2,2-trifluoroacetate production

¹H NMR (400 MHz, CD₂Cl₂) δ12.51 (s, 1H), 10.90 (s, 1H), 10.85 (s, 1H), 10.37 (t, *J* = 1.7 Hz, 1H), 9.53 (t, *J* = 2.1 Hz, 1H), 9.47 (dd, *J* = 2.5, 1.2 Hz, 1H), 6.97 (bs, 1H), 6.58 (s, 3H), 6.06 (bs, 2H), 5.84 (bs, 2H), 5.74 (s, 3H), 4.83 (bs, 2H).

### <Example 12> 1⁵-chloro-3⁶-methoxy-2,5,9-triaza-1(2,4)-pyrimidina-3(1,3)-benzenacyclononapane-4-one;2,2,2-trifluoroacetate production

¹H NMR (400 MHz, CD₂Cl₂) δ10.05 (s, 1H), 9.68 (bs, 1H), 8.18 (d, *J* = 2.1 Hz, 1H), 7.96 (s, 1H), 7.84 (t, *J* = 7.3 Hz, 1H), 7.49 (dd, J = 8.6, 2.1 Hz, 1H), 7.09 (d, *J* = 8.6 Hz, 1H), 6.91 (t, *J* = 5.9 Hz, 1H), 3.95 (s, 3H), 3.43 - 3.34 (m, 2H), 3.30 (q, *J* = 6.5 Hz, 2H), 2.19 (dq, *J* = 14.6, 7.6, 7.1 Hz, 2H).

### <Example 13> 1⁵-chloro-3⁶-ethyl-5-methyl-2,5,9-triaza-1(2,4)-pyrimidina-3(1,3)-benzenacyclononapane-4-one;2,2,2-trifluoroacetate production

¹H NMR (400 MHz, CD₂Cl₂) δ11.35 (s, 1H), 7.78 (s, 1H), 7.61 (d, *J* = 1.7 Hz, 1H), 7.36 (d, *J* = 7.8 Hz, 1H), 7.27 (dd, *J* = 7.9, 1.7 Hz, 1H), 6.85 - 6.80 (m, 1H), 5.09 (bs, 1H), 3.27 - 3.10 (m, 4H), 3.02 (s, 3H), 2.80 (q, *J* = 7.6 Hz, 2H), 2.10 (bs, *J* = 21.3 Hz, 2H), 1.24 (t, *J* = 7.5 Hz, 3H).

### <Example 14> 1⁵-chloro-3⁵-methoxy-5-methyl-2,5,9-triaza-1(2,4)-pyrimidina-3(1,3)-benzenacyclononapane-4-one;2,2,2-trifluoroacetate production

¹H NMR (400 MHz, CD₂Cl₂) δ12.09 (s, 1H), 7.79 (s, 1H), 7.42 (d, *J* = 1.8 Hz, 1H), 6.90 (t, *J* = 1.8 Hz, 1H), 6.82 (t, *J* = 2.2 Hz, 1H), 6.64 - 6.59 (m, 1H), 3.84 (s, 3H), 3.31 (bs, 2H), 3.29 - 3.18 (m, 2H), 3.04 (s, 3H), 2.83 (bs, 1H), 2.15 (bs, 2H).

### <Example 15> 1⁵-chloro-3⁴-methoxy-5-methyl-2,5,9-triaza-1(2,4)-pyrimidina-3(1,3)-benzenacyclononapane-4-one;2,2,2-trifluoroacetate production

¹H NMR (400 MHz, CD₂Cl₂) δ11.27 (bs, 1H), 10.65 (s, 1H), 7.93 (s, 1H), 7.43 (d, *J* = 2.6 Hz, 1H), 7.28 (dd, *J* = 8.8, 2.6 Hz, 1H), 7.00 (d, *J* = 8.8 Hz, 1H), 6.73 (t, *J* = 5.8 Hz, 1H), 3.82 (s, 3H), 3.46 - 3.37 (m, 1H), 3.32 (ddd, *J* = 15.4, 10.0, 5.9 Hz, 1H), 3.15 (s, 3H), 3.07 (td, *J* = 13.5, 5.9 Hz, 2H), 2.48 - 2.28 (m, 1H), 1.84 (q, *J* = 11.8 Hz, 1H).

### <Example 16> 1⁵-chloro-3⁶-methoxy-5-methyl-2,5,10-triaza-1(2,4)-pyrimidina-3(1,3)-benzenacyclodecarphane-4-one;2,2,2-trifluoroacetate production

¹H NMR (400 MHz, CD₂Cl₂) δ10.68 (s, 1H), 10.13 (bs, 1H), 7.78 (s, 1H), 7.55 (d, *J* = 2.1 Hz, 1H), 7.40 (dd, *J* = 8.5, 2.1 Hz, 1H), 7.05 (d, *J* = 8.6 Hz, 1H), 6.57 (t, *J* = 7.0 Hz, 1H), 3.92 (s, 3H), 3.50 (q, *J* = 6.3 Hz, 2H), 3.43 - 3.35 (m, 2H), 3.02 (s, 3H), 1.88 - 1.77 (m, 2H), 1.69 (dt, *J* = 10.8, 5.9 Hz, 2H).

### <Example 17> 1⁵-chloro-3⁵-methoxy-5-methyl-2,5,10-triaza-1(2,4)-pyrimidina-3(1,3)-benzenacyclodecarphane-4-one;2,2,2-trifluoroacetate production

¹H NMR (400 MHz, CD₂Cl₂) δ10.71 (s, 1H), 9.97 (bs, 1H), 7.95 (s, 1H), 7.13 (t, *J* = 1.7 Hz, 1H), 6.93 (dd, *J* = 2.4, 1.3 Hz, 1H), 6.84 (t, *J* = 2.1 Hz, 1H), 6.57 (t, *J* = 7.0 Hz, 1H), 3.83 (s, 3H), 3.51 (q, *J* = 6.2 Hz, 2H), 3.45 (t, *J* = 7.9 Hz, 2H), 3.13 (s, 3H), 1.84 (q, *J* = 9.2, 8.5 Hz, 2H), 1.73 (q, *J* = 9.5, 7.9 Hz, 2H).

### <Example 18> 1⁵-chloro-3⁴-methoxy-5-methyl-2,5,10-triaza-1(2,4)-pyrimidina-3(1,3)-benzenacyclodecarphane-4-one;2,2,2-trifluoroacetate production

¹H NMR (400 MHz, CD₂Cl₂) δ10.34 (s, 1H), 10.33 (bs, 1H), 7.90 (s, 1H), 7.25 (s, 1H), 7.24 (d, *J* = 5.1 Hz, 1H), 6.99 (d, *J* = 9.4 Hz, 1H), 6.54 (t, *J* = 6.7 Hz, 1H), 3.83 (s, 3H), 3.68 (dt, *J* = 14.1, 7.0 Hz, 2H), 3.14 (s, 3H), 3.10 - 2.95 (m, 2H), 1.85 - 1.74 (m, 2H), 1.71 - 1.60 (m, 1H), 1.59 - 1.47 (m, 1H).

### <Example 19> 1⁵-chloro-3⁶-ethyl-5-methyl-2,5,10-triaza-1(2,4)-pyrimidina-3(1,3)-benzenacyclodecarphane-4-one;2,2,2-trifluoroacetate production

¹H NMR (400 MHz, CD₂Cl₂) δ10.70 (bs, 1H), 10.02 (s, 1H), 7.96 (s, 1H), 7.43 (d, *J* = 7.9 Hz, 1H), 7.38 (d, *J* = 1.7 Hz, 1H), 7.35 (dd, *J* = 7.9, 1.8 Hz, 1H), 6.54 (t, *J* = 6.8 Hz, 1H), 3.51 - 3.44 (m, 2H), 3.42 - 3.32 (m, 2H), 3.13 (s, 3H), 2.74 (q, *J* = 7.5 Hz, 2H), 1.84 - 1.75 (m, 2H), 1.73 - 1.64 (m, 2H), 1.22 (t, *J* = 7.6 Hz, 3H).

### <Example 20> 1⁵-chloro-3⁶-methoxy-5,8-dimethyl-2,5,8-triaza-1(2,4)-pyrimidina-3(1,3)-benzenacyclooctaphane-4-one;2,2,2- trifluoroacetate production

¹H NMR (500 MHz, CD₂Cl₂) δ12.21 (bs, 1H), 9.64 (s, 1H), 8.47 (d, *J* = 2.2 Hz, 1H), 7.99 (s, 1H), 7.45 (dd, *J* = 8.5, 2.2 Hz, 1H), 6.99 (d, *J* = 8.5 Hz, 1H), 4.35 (ddd, *J* = 14.2, 11.7, 3.0 Hz, 1H), 4.18 (ddd, *J* = 13.3, 11.7, 3.1 Hz, 1H), 3.90 (s, 3H), 3.67 - 3.59 (m, 1H), 3.58 (s, 3H), 3.23 - 3.11 (m, 1H), 3.16 (s, 3H).

### <Example 21> 1⁵-chloro-5-ethyl-3⁶-methoxy-2,5,8-triaza-1(2,4)-pyrimidina-3(1,3)-benzenacyclooctaphane-4-one;2,2,2-trifluoroacetate production

¹H NMR (500 MHz, CD₂Cl₂) δ11.40 (bs, 1H), 9.75 (s, 1H), 8.64 (d, *J* = 2.2 Hz, 1H), 8.03 (s, 1H), 7.44 (dd, *J* = 8.5, 2.2 Hz, 1H), 6.99 (d, *J* = 8.5 Hz, 1H), 6.64 (t, *J* = 5.0 Hz, 1H), 4.44 - 4.31 (m, 1H), 4.01 - 3.92 (m, 1H), 3.90 (s, 3H), 3.85 - 3.79 (m, 1H), 3.79 - 3.72 (m, 1H), 3.40 (dq, *J* = 14.0, 7.0 Hz, 1H), 3.21 (ddd, *J* = 13.8, 11.0, 6.9 Hz, 1H), 1.29 (t, *J* = 7.1 Hz, 3H).

### <Example 22> 1⁵-chloro-5-isopropyl-3⁶-methoxy-2,5,8-triaza-1(2,4)-pyrimidina-3(1,3)-benzenacyclooctaphane-4-one production

¹H NMR (400 MHz, CD₂Cl₂) δ9.45 (s, 1H), 8.58 (d, *J* = 2.2 Hz, 1H), 7.99 (s, 1H), 7.42 (dd, *J* = 8.6, 2.2 Hz, 1H), 6.98 (d, *J* = 8.6 Hz, 1H), 6.65 (s, 1H), 4.37 (p, *J* = 6.9 Hz, 1H), 4.28 - 4.15 (m, 1H), 3.92 (s, 3H), 3.99 - 3.87 (m, 1H), 3.82 (td, *J* = 10.9, 10.3, 3.8 Hz, 1H), 3.22 (ddd, *J* = 14.1, 11.0, 6.7 Hz, 1H), 1.44 (d, *J* = 7.2 Hz, 3H), 1.42 (d, *J* = 7.1 Hz, 3H).

### <Example 23> 1⁵-chloro-3⁶-methoxy-5,9-dimethyl-2,5,9-triaza-1(2,4)-pyrimidina-3(1,3)-benzenacyclononapane-4-one;2,2,2- trifluoroacetate production

¹H NMR (400 MHz, CD₂Cl₂) δ10.35 (s, 1H), 9.19 (bs, 1H), 7.87 (d, *J* = 2.1 Hz, 1H), 7.84 (s, 1H), 7.36 (dd, *J* = 8.5, 2.1 Hz, 1H), 7.03 (d, *J* = 8.5 Hz, 1H), 3.93 (s, 3H), 3.57 (s, 3H), 3.26 (bs, 4H), 3.06 (s, 3H), 2.08 (bs, 2H).

### <Example 24> 3⁶-methoxy-5-methyl-1⁵-(trifluoromethyl)-2,5,9-triaza-1(2,4)-pyrimidina-3(1,3)-benzenacyclononapane-4-one;2,2,2- trifluoroacetate production

This was synthesized in the same manner as Example 1, except that step 4 was performed in the following manner.

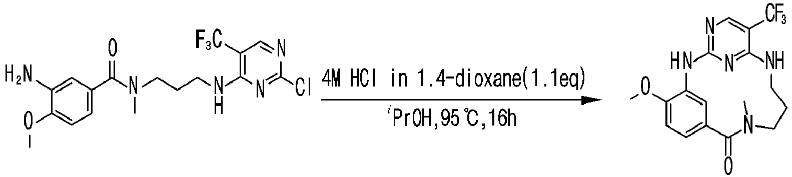

### According to the reaction formula,

3-amino-N-(3-((2-chloro-5-(trifluoromethyl)pyrimidin-4-yl)amino)propyl)-4-methoxy-N-methylbenzamide (335 mg) was dissolved in isopropyl alcohol, and then 1,4-dioxane solution 4.0 M hydrogen chloride (0.22 ml) was added. The mixture was stirred at 95°C for 16 hours. After the reaction was completed, the mixture was concentrated under reduced pressure, extracted with ethyl acetate, and washed with a saturated sodium bicarbonate solution. The organic layer was dried over anhydrous sodium sulfate, filtered, concentrated under reduced pressure, and purified through column chromatography to obtain the target compound (white solid, 269 mg, 80%).

¹H NMR (400 MHz, CD₂Cl₂) δ11.03 (s, 1H), 8.17 (s, 1H), 7.87 (d, *J* = 2.0 Hz, 1H), 7.42 (dd, *J* = 8.5, 2.0 Hz, 1H), 7.06 (d, *J* = 8.5 Hz, 1H), 6.75 - 6.62 (m, 1H), 5.77 (bs, 1H), 3.95 (s, 3H), 3.45 - 3.24 (m, 4H), 3.04 (s, 3H), 2.15 (bs, J = 16.6 Hz, 2H).

### <Example 25> 3⁶-methoxy-5,8-dimethyl-1⁵-(trifluoromethyl)-2,5,8-triaza-1(2,4)-pyrimidina-3(1,3)-benzenacyclooctaphane-4-one;2,2,2-trifluoroacetate production

¹H NMR (400 MHz, CD₂Cl₂) δ10.57 (s, 1H), 10.11 (bs, 1H), 8.52 (d, *J* = 2.2 Hz, 1H), 8.29 (s, 1H), 7.41 (dd, *J* = 8.4, 2.2 Hz, 1H), 6.96 (d, *J* = 8.5 Hz, 1H), 4.40 - 4.29 (m, 1H), 4.18 (t, *J* = 11.7 Hz, 1H), 3.91 (s, 3H), 3.62 (td, *J* = 12.5, 6.0 Hz, 1H), 3.28 (q, *J* = 1.9 Hz, 3H), 3.19 - 3.11 (m, 1H), 3.09 (s, 3H).

### <Example 26> 3⁶-methoxy-5-methyl-1⁵-(trifluoromethyl)-2,5,8-triaza-1(2,4)-pyrimidina-3(1,3)-benzenacyclooctaphane-4-one;2,2,2- trifluoroacetate production

¹H NMR (400 MHz, CD₂Cl₂) δ10.59 (s, 1H), 8.66 (d, *J* = 2.2 Hz, 1H), 8.30 - 8.16 (m, 1H), 8.09 (bs, 1H), 7.42 (dd, *J* = 8.5, 2.1 Hz, 1H), 6.98 (d, *J* = 8.5 Hz, 1H), 6.58 - 6.39 (m, 1H), 4.55 - 4.35 (m, 1H), 3.91 (s, 3H), 3.88 - 3.75 (m, 2H), 3.08 (s, 3H), 3.02 (ddd, *J* = 13.7, 10.9, 7.0 Hz, 1H).

### <Example 27> 1⁵-chloro-3⁶-methoxy-5,10-dimethyl-2,5,10-triaza-1(2,4)-pyrimidina-3(1,3)-benzenacyclodecarphane-4-one;2,2,2- trifluoroacetate production

¹H NMR (400 MHz, CD₂Cl₂) δ12.90 (bs, 1H), 9.08 (s, 1H), 7.85 (s, 1H), 7.65 (d, *J* = 2.1 Hz, 1H), 7.31 (dd, *J* = 8.5, 2.1 Hz, 1H), 7.06 (d, *J* = 8.6 Hz, 1H), 3.89 (s, 3H), 3.62 (t, *J* = 7.0 Hz, 2H), 3.59 (s, 3H), 3.44 (t, *J* = 6.2 Hz, 2H), 3.13 (s, 3H), 1.85 - 1.73 (m, 4H).

### <Example 28> 3⁵-fluoro-3⁶-methoxy-5,9-dimethyl-1⁵-(trifluoromethyl)-2,5,9-triaza-1(2,4)-pyrimidina-3(1,3)-benzenacyclononapane-4-one production

¹H NMR (400 MHz, DMSO) δ9.25 (s, 1H), 8.38 (s, 1H), 7.83 (t, *J* = 1.7 Hz, 1H), 7.00 (dd, *J* = 11.5, 1.9 Hz, 1H), 3.89 (d, *J* = 1.6 Hz, 3H), 3.17 (bs, 4H), 3.12 - 3.07 (m, 3H), 2.93 (s, 3H), 2.11 (bs, 2H).

### <Example 29> 3⁶-methoxy-5,10-dimethyl-1⁵-(trifluoromethyl)-2,5,10-triaza-1(2,4)-pyrimidina-3(1,3)-benzenacyclodecarphane-4-one;2,2, 2-trifluoroacetate production

¹H NMR (400 MHz, CD₂Cl₂) δ13.10 (bs, 1H), 10.39 (s, 1H), 8.30 (s, 1H), 7.64 (d, *J* = 2.1 Hz, 1H), 7.30 (dd, *J* = 8.5, 2.1 Hz, 1H), 7.06 (d, *J* = 8.5 Hz, 1H), 3.91 (s, 3H), 3.68 (t, *J* = 6.7 Hz, 2H), 3.40 - 3.35 (m, 2H), 3.30 (q, *J* = 1.8 Hz, 3H), 3.06 (s, 3H), 1.82 - 1.72 (m, 4H).

### <Example 30> 5,9-dimethyl-3⁶-(trifluoromethoxy)-1⁵-(trifluoromethyl)-2,5,9-triaza-1(2,4)-pyrimidina-3(1,3)-benzenacyclononapane-4-one production

¹H NMR (400 MHz, DMSO) δ9.66 (s, 1H), 8.39 (s, 1H), 8.06 (d, *J* = 2.1 Hz, 1H), 7.44 (dq, *J* = 8.4, 1.6 Hz, 1H), 7.21 (dd, *J* = 8.4, 2.1 Hz, 1H), 3.12 (bs, 4H), 3.09 - 3.08 (m, 3H), 2.95 (s, 3H), 2.10 (bs, 2H).

### <Example 31> 5,9-dimethyl-1⁵,3⁶-bis(trifluoromethyl)-2,5,9-triaza-1(2,4)-pyrimidina-3(1,3)-benzenacyclononapane-4-one production

¹H NMR (400 MHz, DMSO) δ9.16 (s, 1H), 8.38 (s, 1H), 7.93 (d, *J* = 1.6 Hz, 1H), 7.77 (d, *J* = 8.1 Hz, 1H), 7.31 (d, *J* = 8.0 Hz, 1H), 3.18 - 3.01 (m, 5H), 2.96 (s, 3H), 2.95 - 2.90 (m, 2H), 1.99 (bs, 2H).

### <Example 32> 3⁵-methoxy-5,9-dimethyl-1⁵-(trifluoromethyl)-2,5,9-triaza-1(2,4)-pyrimidina-3(1,3)-benzenacyclononapane-4-one;2,2,2-trifluoroacetate production

¹H NMR (400 MHz, CD₂Cl₂) δ12.24 (bs, 1H), 12.05 (s, 1H), 8.25 (s, 1H), 7.42 (t, *J* = 1.7 Hz, 1H), 6.90 (dd, *J* = 2.3, 1.3 Hz, 1H), 6.82 (t, *J* = 2.1 Hz, 1H), 3.83 (s, 3H), 3.32 (d, *J* = 1.8 Hz, 3H), 3.20 (bs, 4H), 3.07 (s, 3H), 2.22 (bs, 2H).

### <Example 33> 3⁶-methoxy-5-methyl-1⁵-(trifluoromethyl)-2,5,10-triaza-1(2,4)-pyrimidina-3(1,3)-benzenacyclodecarphane-4-one;2,2,2- trifluoroacetate production

¹H NMR (400 MHz, CD₂Cl₂) δ10.85 (s, 1H), 9.57 (bs, 1H), 8.13 (s, 1H), 7.55 (d, *J* = 2.1 Hz, 1H), 7.42 (dd, *J* = 8.6, 2.1 Hz, 1H), 7.07 (d, *J* = 8.5 Hz, 1H), 6.48 (d, *J* = 7.0 Hz, 1H), 3.92 (s, 3H), 3.52 (q, J = 6.1 Hz, 2H), 3.41 - 3.36 (m, 2H), 3.05 (s, 3H), 1.88 - 1.76 (m, 2H), 1.69 (q, *J* = 5.4 Hz, 2H).

### <Example 34> 3⁵-fluoro-3⁶-methoxy-5-methyl-1⁵-(trifluoromethyl)-2,5,10-triaza-1(2,4)-pyrimidina-3(1,3)-benzenacyclodecarphane-4-one production

¹H NMR (400 MHz, DMSO) δ 9.11 (s, 1H), 8.17 (d, J = 1.0 Hz, 1H), 7.43 (t, *J* = 1.6 Hz, 1H), 7.17 (t, *J* = 6.2 Hz, 1H), 7.00 (dd, *J* = 11.5, 1.9 Hz, 1H), 3.88 (d, *J* = 1.5 Hz, 3H), 3.33 - 3.26 (m, 4H), 2.90 (s, 3H), 1.72 - 1.63 (m, 2H), 1.55 - 1.47 (m, 2H).

### <Example 35> 5-methyl-3⁶-(trifluoromethoxy)-1⁵-(trifluoromethyl)-2,5,10-triaza-1(2,4)-pyrimidina-3(1,3)-benzenacyclodecarphane-4-one production

¹H NMR (400 MHz, DMSO) δ9.64 (s, 1H), 8.23 (d, *J* = 0.9 Hz, 1H), 7.70 (d, *J* = 2.1 Hz, 1H), 7.50 - 7.37 (m, 2H), 7.24 (dd, *J* = 8.5, 2.1 Hz, 1H), 3.31 - 3.18 (m, 4H), 2.93 (s, 3H), 1.67 (q, *J* = 8.3 Hz, 2H), 1.51 (q, *J* = 5.3, 4.6 Hz, 2H).

The structures of compounds of Examples 1 to 35 according to the present invention are as shown in Table 1.

**[Table 1]**

| Example | Structrue | Example | Strucuture |
|---|---|---|---|
| 1 | 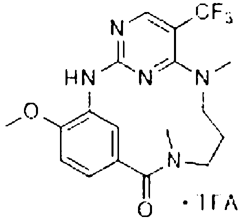 | 19 | 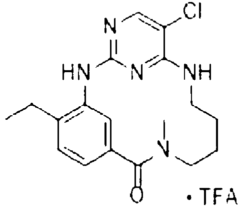 |
| 2 | 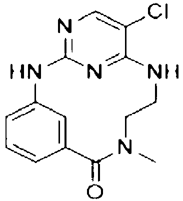 | 20 | 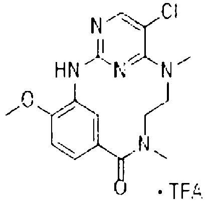 |
| 3 | 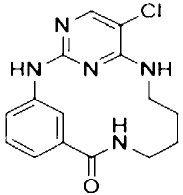 | 21 | 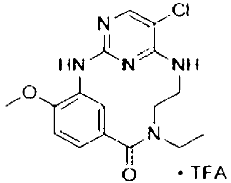 |
| 4 | 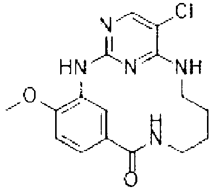 | 22 | 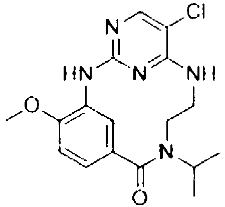 |
| 5 | 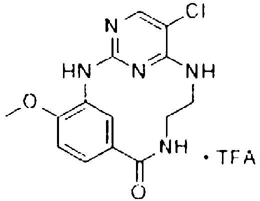 | 23 | 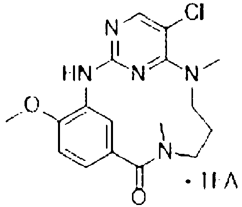 |
| 6 | 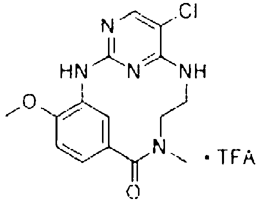 | 24 | 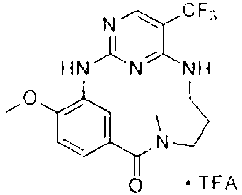 |
| 7 | 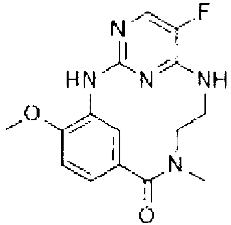 | 25 | 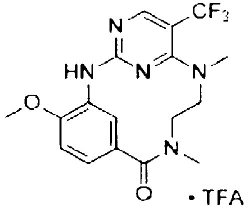 |
| 8 | 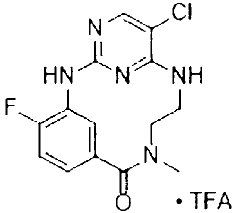 | 26 | 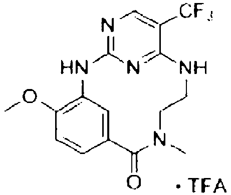 |
| 9 | 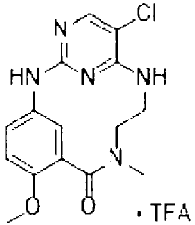 | 27 | 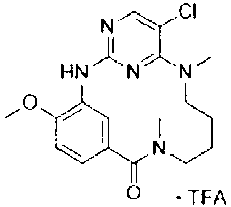 |
| 10 | 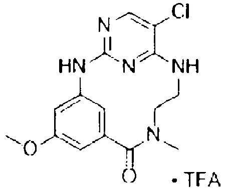 | 28 | 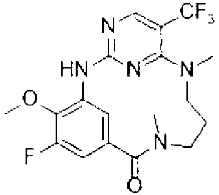 |
| 11 | 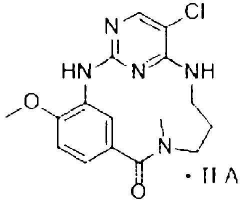 | 29 | 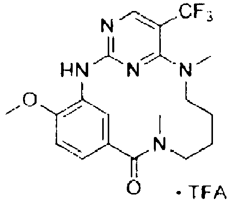 |
| 12 | 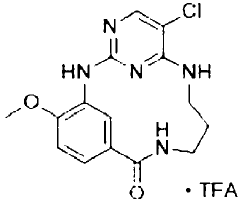 | 30 | 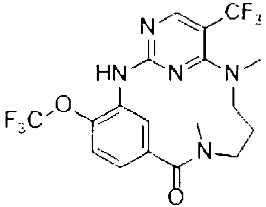 |
| 13 | 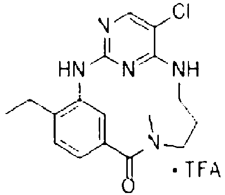 | 31 | 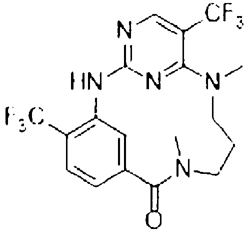 |
| 14 | 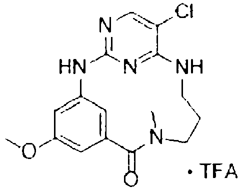 | 32 | 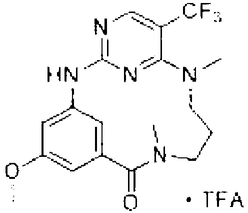 |
| 15 | 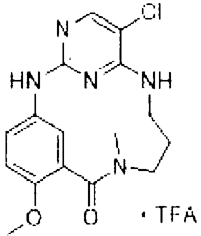 | 33 | 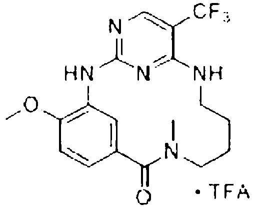 |
| 16 | 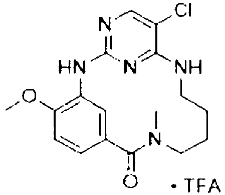 | 34 | 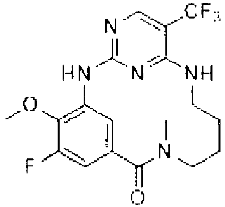 |
| 17 | 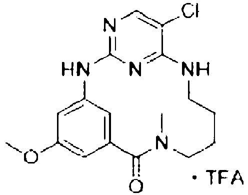 | 35 | 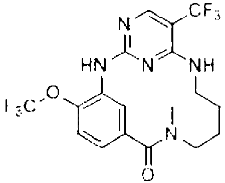 |
| 18 | 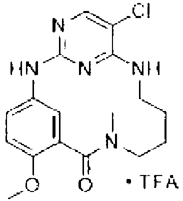 | | |

Compounds of Examples 36 to 41 were prepared similarly to Example 1.<Example 36>
   3⁵-fluoro-3⁶-methoxy-5-methyl-1⁵-(trifluoromethyl)-2,5,9-triaza-1(2,4)-pyrimidina-3(1,3)-benzenacyclonaphane-4-one;
< Example 37>
   3⁵-methoxy-5-methyl-1⁵-(trifluoromethyl)-2,5,9-triaza-1(2,4)-pyrimidina-3(1,3)-benzenacyclonaphane-4-one;
< Example 38>
   5-methyl-3⁶-(oxetane-3-yloxy)-1⁵-(trifluoromethyl)-2,5,9-triaza-1(2,4)-pyrimidina-3(1,3)-benzenacyclonaphane-4-one;
< Example 39>
   3⁶-isopropoxy-5-methyl-1⁵-(trifluoromethyl)-2,5,9-triaza-1(2,4)-pyrimidina-3(1,3)-benzenacyclonaphane-4-one;
< Example 40>
   1⁵-chloro-5-methyl-3⁶-(trifluoromethoxy)-2,5,9-triaza-1(2,4)-pyrimidina-3(1,3)- benzenacyclonaphane-4-one;
< Example 41>
   5-methyl-3⁶-(trifluoromethoxy)-1⁵-(trifluoromethyl)-2,5,9-triaza-1(2,4)-pyrimidina-3(1,3)-benzenacyclonaphane-4-one;
<Example 42>
   1⁵-chloro-3⁶-methoxy-5-methyl-9-oxa-2,5-diaza-1(2,4)-pyrimidina-3(1,3)-benzenacyclonaphane-4-one

This was produced as in Example 1, except for step 1.

### Step 1:

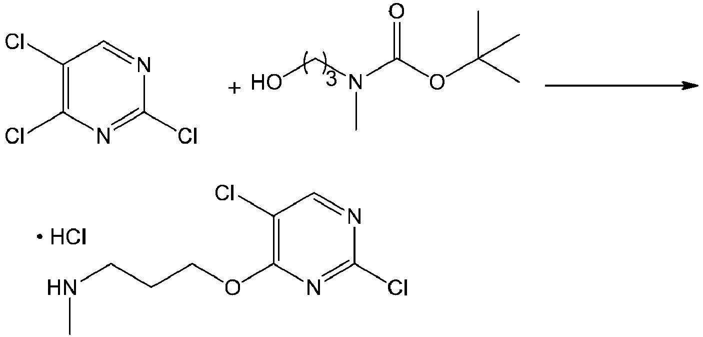

Anhydrous sodium hydride (60% dispersion in mineral oil, 79 mg, 3.3 mmol) was added to a suspension of alcohol (340 mg, 1.1 equivalents) containing Boc- and dissolved in anhydrous tetrahydrofuran at 0°C. After stirring for 5 minutes, 2,4,5-trichloropyrimidine (300 mg, 1.0 equivalents) was slowly added, and the resulting solution was warmed to room temperature and stirred for 1 hour. After completion of the reaction, the reaction mixture was concentrated under reduced pressure, and the mixture was extracted with dichloromethane (50 mL) and washed with brine (20 mL). The organic layer was dried over anhydrous sodium sulfate, filtered, concentrated under reduced pressure, and purified by column chromatography to obtain the desired product (430 mg, 78%).
Examples 43 to 45 were in a similar manner to Example 42 to obtain the target compound.
<Example 43>
   3⁶-methoxy-5-methyl-1⁵-(trifluoromethyl)-9-oxa-2,5-diaza-1(2,4)-pyrimidina-3(1,3)-benzenacyclonaphane-4-one;
< Example 44>
   5-methyl-3⁶-(trifluoromethoxy)-1⁵-(trifluoromethyl)-9-oxa-2,5-diaza-1(2,4)-pyrimidina-3(1,3)-benzenacyclonaphane-4-one;
< Example 45>
   3⁵-fluoro-3⁶-methoxy-5-methyl-1⁵-(trifluoromethyl)-9-oxa-2,5-diaza-1(2,4)-pyrimidina-3(1,3)-benzenacyclonaphane-4-one;
Examples 46 and 47 were in a similar manner to Example 1 to obtain the target compound.
< Example 46>
   3⁶-cyclopropoxy-5-methyl-1⁵-(trifluoromethyl)-2,5,9-triaza-1(2,4)-pyrimidina-3(1,3)-benzenacyclonaphane-4-one;
< Example 47>
   3⁶-cyclopropoxy-5,9-dimethyl-1⁵-(trifluoromethyl)-2,5,9-triaza-1(2,4)-pyrimidina-3(1,3)-benzenacyclonaphane-4-one The compound structures and analytical data of Examples 36 to 47 are as shown in Table 2.

**[Table 2]**

| Example | Strucuture | ¹H NMR |
|---|---|---|
| 36 | 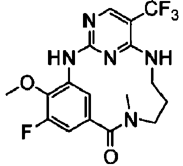 | ¹H NMR (400 MHz, DMSO-*d*₆) δ9.24 (s, 1H), 8.21 (s, 1H), 7.81 (s, 1H), 7.52 (t, *J* = 5.4 Hz, 1H), 6.99 (dd, *J* = 11.6, 1.9 Hz, 1H), 3.90 (d, *J* = 1.6 Hz, 3H), 3.25 - 3.13 (m, 2H), 3.03 (q, *J* = 6.6 Hz, 2H), 2.91 (s, 3H), 1.99 (br, 2H) |
| 37 | 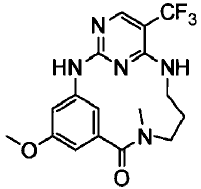 | ¹H NMR (400 MHz, DMSO-*d*₆) δ9.74 (s, 1H), 8.18 (d, *J* = 1.0 Hz, 1H), 7.60 (t, *J* = 1.6 Hz, 1H), 7.47 (t, *J* = 5.3 Hz, 1H), 6.71 (t, *J* = 2.1 Hz, 1H), 6.65 (dd, *J* = 2.5, 1.2 Hz, 1H), 3.76 (s, 3H), 3.25 (br, 2H), 3.03 (br, 2H), 2.92 (s, 3H), 2.01 (br, 2H). |
| 38 | 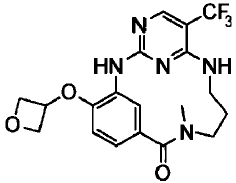 | ¹H NMR (400 MHz, DMSO-*d*₆) δ8.29 (d, *J* = 1.0 Hz, 1H), 8.03 (d, *J* = 2.1 Hz, 1H), 7.60 (t, *J* = 5.5 Hz, 1H), 7.06 (dd, *J* = 8.4, 2.0 Hz, 1H), 6.95 (d, *J* = 8.4 Hz, 1H), 5.06 (s, 1H), 4.78 (br, 1H), 4.23 (s, 1H), 3.65 (dd, *J* = 11.4, 4.6 Hz, 1H), 3.58 - 3.47 (m, 1H), 3.41 - 3.29 (m, 2H), 3.10 (dd, *J* = 13.8, 7.5 Hz, 2H), 3.05 - 2.98 (m, 1H), 2.90 (s, 3H), 2.15 (br, 1H), 1.89 (br, 1H). |
| 39 | 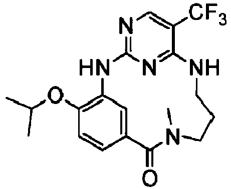 | ¹H NMR (400 MHz, DMSO-*d*₆) δ8.41 (s, 1H), 8.18 (d, *J* = 1.0 Hz, 1H), 8.92 (d, *J* = 2.1 Hz, 1H), 7.49 (t, *J* = 5.3 Hz, 1H), 7.13 (dd, *J* = 8.4, 2.0 Hz, 1H), 7.08 (d, *J* = 8.5 Hz, 1H), 4.69 (hept, *J* = 6.0 Hz, 1H), 3.24 (br, 2H), 3.11 - 2.97 (m, 2H), 2.90 (s, 3H), 2.03 (s, 2H), 1.32 (d, *J* = 6.0 Hz, 6H). |
| 40 | 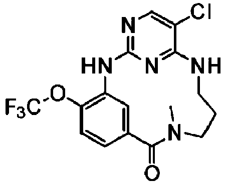 | ¹H NMR (400 MHz, DMSO-*d*₆) δ9.19 (s, 1H), 8.06 (d, *J* = 2.1 Hz, 1H), 7.96 (s, 1H), 7.61 (t, *J* = 5.4 Hz, 1H), 7.39 (dq, *J* = 8.4, 1.6 Hz, 1H), 7.15 (dd, *J* = 8.4, 2.1 Hz, 1H), 3.23 - 3.15 (m, 2H), 3.01 - 2.95 (m, 2H), 2.93 (s, 3H), 1.99 (br, 2H). |
| 41 | 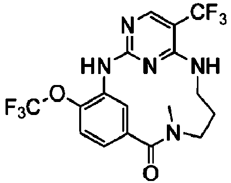 | ¹H NMR (400 MHz, DMSO-*d*₆) δ9.65 (s, 1H), 8.21 (s, 1H), 8.04 (d, *J* = 2.1 Hz, 1H), 7.53 (t, *J* = 5.4 Hz, 1H), 7.42 (dd, *J* = 8.4, 1.7 Hz, 1H), 7.20 (dd, *J* = 8.5, 2.0 Hz, 1H), 3.17 - 3.08 (m, 2H), 3.03 - 2.95 (m, 3H), 2.93 (s, 3H), 1.99 (br, 2H). |
| 42 | 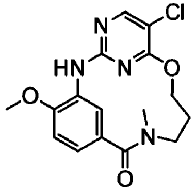 | ¹H NMR (400 MHz, DMSO-*d*₆) δ8.89 (s, 1H), 8.28 (s, 1H), 7.88 (d, *J* = 2.1 Hz, 1H), 7.15 (dd, *J* = 8.4, 2.1 Hz, 1H), 7.09 (d, *J* = 8.4 Hz, 1H), 4.12 (t, *J* = 7.0 Hz, 2H), 3.86 (s, 3H), 3.23 (br, 2H), 2.90 (s, 3H), 2.25 (br, 2H). |
| 43 | 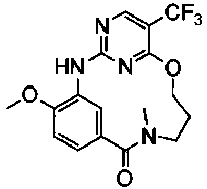 | ¹H NMR (400 MHz, DMSO-*d*₆) δ9.50 (s, 1H), 8.49 (d, J= 1.0 Hz, 1H), 7.88 (d, *J* = 2.1 Hz, 1H), 7.19 (dd, *J* = 8.4, 2.1 Hz, 1H), 7.12 (d, 7 = 8.5 Hz, 1H), 4.13 (t, *J* = 7.0 Hz, 2H), 3.87 (s, 3H), 3.20 (br, 2H), 2.90 (s, 3H), 2.25 (br, 2H). |
| 44 | 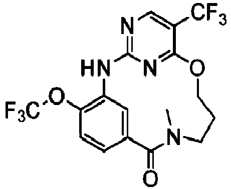 | ¹HNMR (400 MHz, Methylene Chloride-*d*₂) δ8.46 (s, 1H), 8.07 (d, *J* = 1.9 Hz, 1H), 7.87 (s, 1H), 7.39 (dd, *J* = 8.5, 1.6 Hz, 1H), 7.36 (dd, *J* = 8.5, 1.9 Hz, 1H), 4.25 (t, *J* = 7.1 Hz, 2H), 3.35 (br, 2H), 3.03 (s, 3H), 2.34 (br, 2H). |
| 45 | 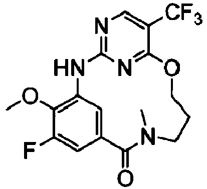 | ¹H NMR (400 MHz, DMSO-*d*₆) δ9.98 (s, 1H), 8.54 (d, *J* = 1.0 Hz, 1H), 7.76 (s, 1H), 7.06 (dd, *J* = 11.5, 1.9 Hz, 1H), 4.16 (t, *J* = 7.0 Hz, 2H), 3.92 (d, *J* = 1.6 Hz, 3H), 3.20 (br, 2H), 2.24 (br, 2H). |
| 46 | 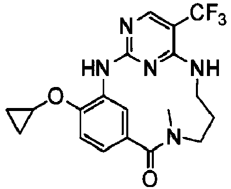 | ¹H NMR (400 MHz, DMSO-*d*₆) δ8.66 (s, 1H), 8.18 (d, *J* = 1.0 Hz, 1H), 7.97 (d, *J* = 2.1 Hz, 1H), 7.54 (t, *J* = 5.4 Hz, 1H), 7.35 (d, *J* = 8.4 Hz, 1H), 7.17 (dd, *J* = 8.4, 2.1 Hz, 1H), 3.94 (tt, *J* = 6.0, 3.1 Hz, 1H), 3.22 (br, 2H), 3.08 - 2.95 (m, 2H), 2.02 (br, 2H), 0.85 - 0.73 (m, 4H), |
| 47 | 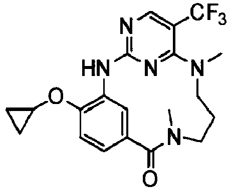 | ¹HNMR (400 MHz, Methylene Chloride-*d*₂) δ8.31 (s, 1H), 8.28 (d, *J* = 2.1 Hz, 1H), 7.58 (br, 1H), 7.33 (d, *J* = 8.4 Hz, 1H), 7.29 (dd, *J* = 8.4, 2.1 Hz, 1H), 3.88 - 3.81 (m, 1H), 3.31 (br, 4H), 3.18 (d, *J* = 2.1 Hz, 3H), 3.02 (s, 3H), 2.24 (br, 2H), 0.88 - 0.81 (m, 4H). |

**<Experimental Example 1> Evaluation of LRRK2 inhibitory activity** To evaluate the inhibitory activity of wild type and G2019S mutant of LRRK2 (leucine-rich repeat kinase 2), the "HotSpot" assay platform was used at Reaction Biology Corporation. The experimental method was as follows.

Specific kinase/substrate pairs were prepared in reaction buffer (20 mM Hepes pH 7.5, 10 mM MgCl2, 1 mM EGTA, 0.02% Brij35, 0.02 mg/ml BSA, 0.1 mM Na3VO4, 2 mM DTT, 1% DMSO) along with the required cofactors. The compounds were transferred to the reaction, and after 20 min, a mixture of ATP (Sigma) and ³³P ATP (PerkinElmer) was added to a final concentration of 10 µM. The reaction was carried out at 25°C for 120 min and then dropped onto P81 ion exchange filter paper (Whatman). The filters were washed extensively in 0.75% phosphoric acid to remove unbound phosphate. After subtracting the background value from the control reaction containing inactive enzyme, the kinase activity data were expressed as the percentage of kinase activity remaining in the test sample compared to the solvent (dimethyl sulfoxide) reaction. IC₅₀ values were obtained using Prism (GraphPad Software). The results are shown in Table 3.

**[Table 3]**

| Example | Wild type (IC₅₀, nM) | G2019S (IC₅₀, nM) | Example | Wild type (IC₅₀, nM) | G2019S (IC₅₀, nM) |
|---|---|---|---|---|---|
| 1 | 0.279 | <0.254 | 25 | 62.9 | 25.7 |
| 2 | 17 | 19.3 | 26 | 6.73 | 3.38 |
| 3 | 15.9 | 13.1 | 27 | 5.42 | 2 |
| 4 | 16.5 | 15 | 28 | 2.72 | 2.47 |
| 5 | 6.71 | 8.1 | 29 | 1.79 | 0.72 |
| 6 | 1.87 | 4 | 30 | 1.73 | 0.574 |
| 7 | 35.5 | 34.1 | 31 | 58.1 | 33.4 |
| 8 | 50.4 | 51.2 | 32 | 2.89 | 0.471 |
| 9 | 91.1 | 79.7 | 33 | 1.41 | 1.02 |
| 10 | 15.8 | 16 | 34 | 8.21 | 5.30 |
| 11 | 4.51 | 4.33 | 35 | 3.72 | 1.80 |
| 12 | 5.51 | 5.4 | 36 | 8.54 | 3.48 |
| 13 | 4.98 | 4.94 | 37 | 9.23 | 4.93 |
| 14 | 9.24 | 9.38 | 38 | 22.2 | 46.7 |
| 15 | 26.6 | 23.3 | 39 | 4.17 | 1.84 |
| 16 | 6.62 | 7.12 | 40 | 5.47 | 3.81 |
| 17 | 35.1 | 28.6 | 41 | 4.82 | 3.08 |
| 18 | 142 | 145 | 42 | 5.91 | 5.14 |
| 19 | 13.4 | 6.86 | 43 | 4.87 | 2.07 |
| 20 | 3.44 | 3.51 | 44 | 16.2 | 10.5 |
| 21 | 11.1 | 3.09 | 45 | 10.2 | 4.8 |
| 22 | 5.06 | 0.98 | 46 | 6.8 | 2.5 |
| 23 | 0.667 | <0.254 | 47 | 6.3 | 2.7 |
| 24 | 0.642 | <0.254 | | | |

Referring to Table 2, the macrocyclic pyrimidine derivative of the present invention may be confirmed to have excellent inhibitory activity against the wild type and G2019S mutant type of LRRK2 protein. Therefore, the compounds according to the present invention are expected to be effective in preventing or treating related diseases caused by overactivation of the wild type LRRK2 protein or the G2019S mutant LRRK2 protein.

### <Experimental Example 2> Evaluation of Blood-Brain Barrier (BBB) permeability

To evaluate the blood-brain barrier permeability, the following experiment was conducted.

The test substance was prepared at a dosage of 5 mg/kg, and after oral administration, the animals were sacrificed at 1 and 3 hours, and blood was collected from the heart and centrifuged to prepare plasma samples. To remove the remaining blood in the body, the brain was removed after cardiac perfusion using 20 ml of saline containing 10 U/ml heparin. The brain tissue was homogenized by adding 3 times the weight of PBS buffer. LC-MS/MS (Agilent, Santa Clara, CA, USA) was used for quantitative analysis of the prepared plasma and brain tissue. The separation of substances in LC used a mobile phase consisting of 20% water with 80% acetonitrile and 0.1% formic acid. The total run time per substance was 3 minutes, and the flow rate was 0.3 ml/min. The results are shown in Table 4.

**[Table 4]**

| Example | BBB permeability (%) |
|---|---|
| 1 | 68 |
| 6 | 6 |
| 8 | 46 |
| 10 | 9 |
| 11 | 12 |
| 13 | 12 |
| 14 | 56 |
| 15 | 11 |
| 16 | 12 |
| 17 | 38 |
| 24 | 30 |
| 26 | 35 |
| 32 | 51 |

Looking at Table 3, the average BBB permeability of the example is about 30%, and in particular, in the case of Example 1, the BBB permeability is very high at 68%. Therefore, it is expected that the compound according to the present invention will be effective in preventing or treating neurodegenerative diseases by efficiently passing through the BBB.

## Claims

1. A compound represented by Formula 1, a solvate thereof, a hydrate thereof, or a pharmaceutically acceptable salt thereof: 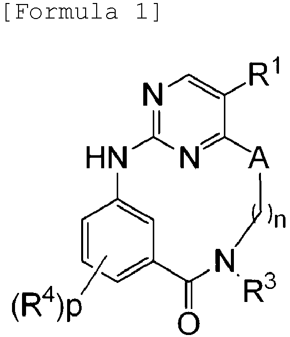
wherein, in Formula 1,
A is O or N(R²),
R¹ is hydrogen, halogen or C₁₋₁₀ alkyl unsubstituted or substituted with halogen;
R² and R³ are each independently hydrogen or unsubstituted C₁₋₁₀ alkyl;
p is 0 to 3;
R⁴ is independently selected from the group consisting of halogen, C₁₋₁₀ alkyl, or OR^{4a}, wherein R^{4a} is hydrogen, C₁₋₁₀ alkyl unsubstituted or substituted with halogen, C₃₋₇ cycloalkyl, or 3 to 7 membered heterocycloalkyl; and
n is 1 to 5.

2. The compound, the solvate thereof, the hydrate thereof, or the pharmaceutically acceptable salt thereof of claim 1,
wherein R¹ is halogen, or C₁₋₆ alkyl unsubstituted or substituted with 1 to 5 halogens;
R² and R³ are each independently hydrogen, or C₁₋₆ alkyl;
p is 0 to 2;
R⁴ is independently selected from the group consisting of hydrogen, halogen, C₁₋₆ alkyl or OR^{4a}, wherein R^{4a} is C₁₋₆ alkyl unsubstituted or substituted with 1 to 5 halogens, C₃₋₆ cycloalkyl, or 3 to 6 membered heterocycloalkyl; and
n is 1 to 5.

3. The compound, the solvate thereof, the hydrate thereof, or the pharmaceutically acceptable salt thereof of claim 1,
wherein R¹ is halogen, or C₁₋₃ alkyl substituted with 1 to 3 halogens;
R² and R³ are each independently hydrogen, or C₁₋₃ alkyl;
p is 0 to 2;
R⁴ is independently selected from the group consisting of halogen, C₁₋₃ alkyl, or OR^{4a},
wherein R^{4a} is C₁₋₃ alkyl unsubstituted or substituted with 1 to 3 halogens, C₃₋₆ cycloalkyl or 3 to 6 membered heterocycloalkyl; and
n is 1 to 5.

4. The compound, the solvate thereof, the hydrate thereof, or the pharmaceutically acceptable salt thereof of claim 1,
wherein R¹ is -Cl, -F, or CF₃;
R² and R³ are each independently hydrogen, or C₁₋₃ alkyl;
p is 0 to 2;
R⁴ is independently selected from the group consisting of -F, C₁₋₃ alkyl, or OR^{4a},
wherein R^{4a} is C₁₋₃ alkyl unsubstituted or substituted with 1 to 3 halogens, C₃₋₆ cycloalkyl, or 3 to 6 membered heterocycloalkyl containing one O atom; and
n is 1 to 4.

5. The compound, the solvate thereof, the hydrate thereof, or the pharmaceutically acceptable salt thereof of claim 1,
Wherein R¹ is -Cl, -F, or -CF₃;
R² and R³ are each independently hydrogen, methylethyl or isopropyl;
p is 0 to 2;
R⁴ is independently -F, ethyl, -CF₃, methoxy, isopropoxy, -OCF₃, cyclopropoxy, oxetane-3-yloxy; and
n is 2 to 4.

6. The compound, the solvate thereof, the hydrate thereof, or the pharmaceutically acceptable salt thereof of claim 1, wherein the compound represented by Formula 1 is one selected from the following group of compounds:
<1> 3⁶-methoxy-5,9-dimethyl-1⁵-(trifluoromethyl)-2,5,9-triaza-1(2,4)-pyrimidina-3(1,3)-benzenacyclononaphan-4-one;2,2,2-trifluoroacetate;
<2> 1⁵-chloro-5-methyl-2,5,8-triaza-1(2,4)-pyrimidina-3(1,3)-benzenacyclooctaphane-4-one;
<3> 1⁵-chloro-2,5,10-triaza-1(2,4)-pyrimidina-3(1,3)-benzenacyclodecarphane-4-one;
<4> 1⁵-chloro-3⁶-methoxy-2,5,10-triaza-1(2,4)-pyrimidina-3(1,3)-benzenacyclodecarphane-4-one;
<5> 1⁵-chloro-3⁶-methoxy-2,5,8-triaza-1(2,4)-pyrimidina-3(1,3)-benzenacyclooctaphane-4-one;
<6> 1⁵-chloro-3⁶-methoxy-5-methyl-2,5,8-triaza-1(2,4)-pyrimidina-3(1,3)-benzenacyclooctaphane-4-one;2,2,2-trifluoroacetate;
<7> 1⁵-fluoro-3⁶-methoxy-5-methyl-2,5,8-triaza-1(2,4)-pyrimidina-3(1,3)-benzenacyclooctaphane-4-one;
<8> 1⁵-chloro-3⁶-fluoro-5-methyl-2,5,8-triaza-1(2,4)-pyrimidina-3(1,3)-benzenacyclooctaphane-4-one;2,2,2-trifluoroacetate;
<9> 1⁵-chloro-3⁴-methoxy-5-methyl-2,5,8-triaza-1(2,4)-pyrimidina-3(1,3)-benzenacyclooctaphane-4-one;2,2,2-trifluoroacetate;
<10> 1⁵-chloro-3⁵-methoxy-5-methyl-2,5,8-triaza-1(2,4)-pyrimidina-3(1,3)-benzenacyclooctaphane-4-one;2,2,2-trifluoroacetate;
<11> 1⁵-chloro-3⁶-methoxy-5-methyl-2,5,9-triaza-1(2,4)-pyrimidina-3(1,3)-benzenacyclononapane-4-one;2,2,2-trifluoroacetate;
<12> 1⁵-chloro-3⁶-methoxy-2,5,9-triaza-1(2,4)-pyrimidina-3(1,3)-benzenacyclononapane-4-one;2,2,2-trifluoroacetate;
<13> 1⁵-chloro-3⁶-ethyl-5-methyl-2,5,9-triaza-1(2,4)-pyrimidina-3(1,3)-benzenacyclononapane-4-one;2,2,2-trifluoroacetate;
<14> 1⁵-chloro-3⁵-methoxy-5-methyl-2,5,9-triaza-1(2,4)-pyrimidina-3(1,3)-benzenacyclononapane-4-one;2,2,2-trifluoroacetate;
<15> 1⁵-chloro-3⁴-methoxy-5-methyl-2,5,9-triaza-1(2,4)-pyrimidina-3(1,3)-benzenacyclononapane-4-one;2,2,2-trifluoroacetate;
<16> 1⁵-chloro-3⁶-methoxy-5-methyl-2,5,10-triaza-1(2,4)-pyrimidina-3(1,3)-benzenacyclodecarphane-4-one;2,2,2-trifluoroacetate;
<17> 1⁵-chloro-3⁵-methoxy-5-methyl-2,5,10-triaza-1(2,4)-pyrimidina-3(1,3)-benzenacyclodecarphane-4-one;2,2,2-trifluoroacetate;
<18> 1⁵-chloro-3⁴-methoxy-5-methyl-2,5,10-triaza-1(2,4)-pyrimidina-3(1,3)-benzenacyclodecarphane-4-one;2,2,2-trifluoroacetate;
<19> 1⁵-chloro-3⁶-ethyl-5-methyl-2,5,10-triaza-1(2,4)-pyrimidina-3(1,3)-benzenacyclodecarphane-4-one;2,2,2-trifluoroacetate;
<20> 1⁵-chloro-3⁶-methoxy-5,8-dimethyl-2,5,8-triaza-1(2,4)-pyrimidina-3(1,3)-benzenacyclooctaphane-4-one;2,2,2-trifluoroacetate;
<21> 1⁵-chloro-5-ethyl-3⁶-methoxy-2,5,8-triaza-1(2,4)-pyrimidina-3(1,3)-benzenacyclooctaphane-4-one;2,2,2-trifluoroacetate;
<22> 1⁵-chloro-5-isopropyl-3⁶-methoxy-2,5,8-triaza-1(2,4)-pyrimidina-3(1,3)-benzenacyclooctaphane-4-one;
<23> 1⁵-chloro-3⁶-methoxy-5,9-dimethyl-2,5,9-triaza-1(2,4)-pyrimidina-3(1,3)-benzenacyclononapane-4-one;2,2,2-trifluoroacetate;
<24> 3⁶-methoxy-5-methyl-1⁵-(trifluoromethyl)-2,5,9-triaza-1(2,4)-pyrimidina-3(1,3)-benzenacyclononapane-4-one;2,2,2- trifluoroacetate;
<25> 3⁶-methoxy-5,8-dimethyl-1⁵-(trifluoromethyl)-2,5,8-triaza-1(2,4)-pyrimidina-3(1,3)-benzenacyclooctaphane-4-one;2,2, 2-trifluoroacetate;
<26> 3⁶-methoxy-5-methyl-1⁵-(trifluoromethyl)-2,5,8-triaza-1(2,4)-pyrimidina-3(1,3)-benzenacyclooctaphane-4-one;2,2,2- trifluoroacetate;
<27> 1⁵-chloro-3⁶-methoxy-5,10-dimethyl-2,5,10-triaza-1(2,4)-pyrimidina-3(1,3)-benzenacyclodecarphane-4-one;2,2,2-trifluoroacetate;
<28> 3⁵-fluoro-3⁶-methoxy-5,9-dimethyl-1⁵-(trifluoromethyl)-2,5,9-triaza-1(2,4)-pyrimidina-3(1,3)-benzenacyclononapane-4-one;
<29> 3⁶-methoxy-5,10-dimethyl-1⁵-(trifluoromethyl)-2,5,10-triaza-1(2,4)-pyrimidina-3(1,3)-benzenacyclodecarphane-4-one;2,2, 2-trifluoroacetate;
<30> 5,9-dimethyl-3⁶-(trifluoromethoxy)-1⁵-(trifluoromethyl)-2,5,9-triaza-1(2,4)-pyrimidina-3(1,3)-benzenacyclononapane-4-one;
<31> 5,9-dimethyl-1⁵,3⁶-bis(trifluoromethyl)-2,5,9-triaza-1(2,4)-pyrimidina-3(1,3)-benzenacyclononapane-4-one;
<32> 3⁵-methoxy-5,9-dimethyl-1⁵-(trifluoromethyl)-2,5,9-triaza-1(2,4)-pyrimidina-3(1,3)-benzenacyclononaphan-4-one;2,2,2-trifluoroacetate;
<33> 3⁶-methoxy-5-methyl-1⁵-(trifluoromethyl)-2,5,10-triaza-1(2,4)-pyrimidina-3(1,3)-benzenacyclodecarphane-4-one;2,2,2-trifluoroacetate;
<34> 3⁵-fluoro-3⁶-methoxy-5-methyl-1⁵-(trifluoromethyl)-2,5,10-triaza-1(2,4)-pyrimidina-3(1,3)-benzenacyclodecarphane-4-one; and
<35> 5-methyl-3⁶-(trifluoromethoxy)-1⁵-(trifluoromethyl)-2,5,10-triaza-1(2,4)-pyrimidina-3(1,3)-benzenacyclodecarphane-4-one;
<36> 3⁵-fluoro-3⁶-methoxy-5-methyl-1⁵-(trifluoromethyl)-2,5,9-triaza-1(2,4)-pyrimidina-3(1,3)-benzenacyclonaphane-4-one;
<37> 3⁵-methoxy-5-methyl-1⁵-(trifluoromethyl)-2,5,9-triaza-1(2,4)-pyrimidina-3(1,3)-benzenacyclonaphane-4-one;
<38> 5-methyl-3⁶-(oxetane-3-yloxy)-1⁵-(trifluoromethyl)-2,5,9-triaza-1(2,4)-pyrimidina-3(1,3)-benzenacyclonaphane-4-one;
<39> 3⁶-isopropoxy-5-methyl-1⁵-(trifluoromethyl)-2,5,9-triaza-1(2,4)-pyrimidina-3(1,3)-benzenacyclonaphane-4-one;
<40> 1⁵-chloro-5-methyl-3⁶-(trifluoromethoxy)-2,5,9-triaza-1(2,4)-pyrimidina-3(1,3)- benzenacyclonaphane-4-one;
<41> 5-methyl-3⁶-(trifluoromethoxy)-1⁵-(trifluoromethyl)-2,5,9-triaza-1(2,4)-pyrimidina-3(1,3)-benzenacyclonaphane-4-one;
<42> 1⁵-chloro-3⁶-methoxy-5-methyl-9-oxa-2,5-diaza-1(2,4)-pyrimidina-3(1,3)-benzenacyclonaphane-4-one;
<43> 3⁶-methoxy-5-methyl-1⁵-(trifluoromethyl)-9-oxa-2,5-diaza-1(2,4)-pyrimidina-3(1,3)-benzenacyclonaphane-4-one;
<44> 5-methyl-3⁶-(trifluoromethoxy)-1⁵-(trifluoromethyl)-9-oxa-2,5-diaza-1(2,4)-pyrimidina-3(1,3)-benzenacyclonaphane-4-one;
<45> 3⁵-fluoro-3⁶-methoxy-5-methyl-1⁵-(trifluoromethyl)-9-oxa-2,5-diaza-1(2,4)-pyrimidina-3(1,3)-benzenacyclonaphane-4-one;
<46> 3⁶-cyclopropoxy-5-methyl-1⁵-(trifluoromethyl)-2,5,9-triaza-1(2,4)-pyrimidina-3(1,3)-benzenacyclonaphane-4-one; and
<47> 3⁶-cyclopropoxy-5,9-dimethyl-1⁵-(trifluoromethyl)-2,5,9-triaza-1(2,4)-pyrimidina-3(1,3)-benzenacyclonaphane-4-one.

7. A method for preparing a compound represented by Formula 1, comprising, as shown in Reaction Formula 1 below, preparing a compound represented by Formula 1 by cyclizing a compound represented by Formula 1A: 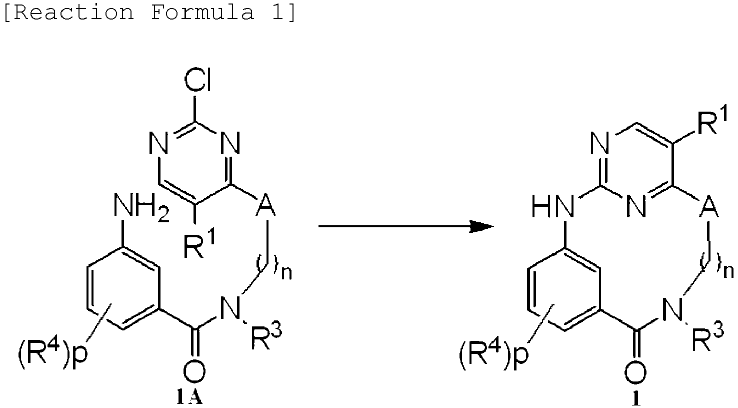
in Reaction Formula 1,
A, R¹, R³, R⁴, p and n are as defined in Formula 1 of claim 1.

8. A pharmaceutical composition for preventing or treating neurodegenerative disease, containing as an active ingredient a compound represented by Formula 1 of claim 1, a solvate thereof, a hydrate thereof, or a pharmaceutically acceptable salt thereof.

9. The pharmaceutical composition of claim 8, wherein the neurodegenerative disease includes Parkinson's disease, Alzheimer's disease, Huntington's disease, Pick's disease, Amyotrophic lateral sclerosis, Prion disease, Motor neuron disease, Spinocerebellar ataxia, Spinal muscular atrophy, Creutzfeldt-Jakob disease, or Alcohol related dementia.

10. The pharmaceutical composition of claim 8, wherein the compound inhibits LRRK2 (leucine-rich repeat kinase 2) activity.

11. A pharmaceutical composition for preventing or alleviating neurodegenerative disease, containing, as an active ingredient, a compound represented by Formula 1 of claim 1, a solvate thereof, a hydrate thereof, or a pharmaceutically acceptable salt thereof.
